# EUROPEAN PATENT SPECIFICATION

(11) **EP 1 037 649 B1**
(45) Date of publication and mention of the grant of the patent: **30.09.2009**
(21) Application number: 98963173.4
(22) Date of filing: 14.12.1998
(51) Int. Cl.: A61K 47/48, A61P 35/00

(54) **POLYMERIC PRODRUGS OF AMINO- AND HYDROXYL-CONTAINING BIOACTIVE AGENTS**
POLYMERE VORSTUFENWIRKSTOFFE VON AMINO-UND HYDROXYL-ENTHALTENDEN BIOAKTIVEN AGENTIEN
PRODROGUES POLYMERIQUES D'AGENTS BIOACTIFS CONTENANT AMINE OU HYDROXY

(30) Priority: 17.12.1997 US 992435; 30.10.1998 US 183557
(43) Date of publication of application: 27.09.2000
(60) Divisional application: 07117973.3
(73) Proprietor: ENZON, INC., Piscataway, NJ 08854-3998 (US)
(72) Inventor: GREENWALD, Richard, B., Somerset, NJ 08873 (US); PENDRI, Annapurna, Matawan, NJ 07747 (US); CHOE, Yun, H., Piscataway, NJ 08854 (US); ZALIPSKY, Samuel, Redwood City, CA 94061 (US)
(74) Representative: Prins, Adrianus Willem
(86) International application number: PCT/US1998/026565
(87) International publication number: WO 1999/030727

(56) References cited:
- EP-A- 0 648 503
- US-A- 5 093 531
- US-A- 5 349 001
- US-A- 5 605 976
- SENTER P D ET AL: "POLY(ETHYLENE GLYCOL)-DOXURUBICIN CONJUGATES CONTAINING LACTAMASE-SENSITIVE LINKERS" BIOCONJUGATE CHEMISTRY, AMERICAN CHEMICAL SOCIETY, WASHINGTON, US, vol. 6, no. 4, 1 July 1995 (1995-07-01), pages 389-394, XP000517227 ISSN: 1043-1802
- PATENT ABSTRACTS OF JAPAN vol. 0184, no. 54 (P-1791), 24 August 1994 (1994-08-24) & JP 6 148780 A (KONICA CORP), 27 May 1994 (1994-05-27)
- GREENWALD R B: "Drug delivery systems: Anticancer prodrugs and their polymeric conjugates" EXPERT OPINION ON THERAPEUTIC PATENTS 1997 UNITED KINGDOM, vol. 7, no. 6, 6 June 1997 (1997-06-06), pages 601-609, XP002296827 ISSN: 1354-3776
- BUNDGAARD H: "THE DOUBLE PRODRUG CONCEPT AND ITS APPLICATIONS" ADVANCED DRUG DELIVERY REVIEWS, AMSTERDAM, NL, vol. 3, 1989, pages 39-65, XP000606520 ISSN: 0169-409X
- GESSON JP ET AL: "Prodrugs of anthracyclines for chemotherapy via enzyme-monoclonal antibody conjugates" ANTI-CANCER DRUG DESIGN, BASINGSTOKE, GB, October 1994 (1994-10), XP002076228 ISSN: 0266-9536
- GREENWALD R.B. ET AL: 'Drug delivery systems employing 1,4- or 1,6-elimination: poly(ethylene glycol) prodrugs of amine-containing compounds' J.MED.CHEM vol. 42, 1999, pages 3657 - 3667

## Description

### TECHNICAL FIELD

The present invention relates to double prodrugs. In particular, the invention relates to polymeric-based double prodrugs having reversible linkages involving amino and hydroxyl moieties of chemical compounds and biologically active materials such as enzymes, proteins and the like.

### BACKGROUND OF THE INVENTION

Over the years, several methods of administering biologically-effective materials to mammals have been proposed. Many medicinal agents are available as water-soluble salts and can be included in pharmaceutical formulations relatively easily. Problems arise when the desired medicinal agent is either insoluble in aqueous fluids or is rapidly degraded *in vivo.* For example, alkaloids are often especially difficult to solubilize.

One way to solubilize medicinal agents is to include them as part of a soluble prodrug. Prodrugs include chemical derivatives of a biologically-active parent compound which, upon administration, eventually liberate the parent compound *in vivo.* Prodrugs allow the artisan to modify the onset and/or duration of action of an agent *in vivo* and can modify the transportation, distribution or solubility of a drug in the body. Furthermore, prodrug formulations often reduce the toxicity and/or otherwise overcome difficulties encountered when administering pharmaceutical preparations. Typical examples of prodrugs include organic phosphates or esters of alcohols or thioalcohols. See Remington's Pharmaceutical Sciences, 16th Ed., A. Osol, Ed. (1980).

Prodrugs are often biologically inert, or substantially inactive, forms of the parent or active compound. The rate of release of the active drug, i.e. the rate of hydrolysis, is influenced by several factors but especially by the type of bond joining the parent drug to the modifier. Care must be taken to avoid preparing prodrugs which are eliminated through the kidney or reticular endothelial system, etc. before a sufficient amount of hydrolysis of the parent compound occurs. By incorporating a polymer as part of the prodrug system, one can increase the circulating half-life of the drug. However, in some situations such as with alkaloids, it has been determined that when only one or two polymers of less than about 10,000 daltons are conjugated thereto, the resulting conjugates are rapidly eliminated *in vivo* especially if a somewhat hydrolysis-resistant linkage is used. In fact, such conjugates are so rapidly cleared from the body that even if a hydrolysis-prone ester linkage is used, not enough of the parent molecule is regenerated *in vivo.* This is often not a concern with moieties such as proteins, enzymes and the like even when hydrolysis-resistant linkages are used. In those cases multiple polymer strands, each having a molecular weight of about 2-5 kDa, are used to further increase the molecular weight and circulating half-life.

Although the above-mentioned concept of prodrug-based delivery systems has proven to be useful in many instances, there are nonetheless situations where alternatives are desired. For example, Bundgaard in "The Double Prodrug Concept and Its Applications" in Advanced Drug Delivery Reviews, 3 (1989) 39-65, pointed out that in many cases it is difficult to obtain a prodrug which has the proper combination of adequate stability *in vitro* and high susceptibility to regenerate the parent drug *in vivo.* As pointed out by Bundgaard, a promising means of overcoming some of the previously encountered shortcomings involves the use of cascade latentiation or "pro-prodrugs". In such systems, the hydrolytic reaction sequence involves a first step which usually is an enzymatic cleavage and the second involves a non-enzymatic hydrolysis that occurs only after the first has taken place.

Senter P.D. et al., Bioconjugate Chemistry (1995) Vol. 6, No. 4, 389-394, describes poly(ethylene glycol)-doxorubicin conjugates containing a cephalosporin moiety as a β-lactamase sensitive linker.

JP 6148780 describes an aromatic compound R¹-Ar-CH(OH)-R² for use in a silver halide photographic sensitive material, wherein Ar is an arylene radical, at least one of R¹ and R² comprises at least three repetitive alkyleneoxy units and the other may be a hydrogen atom. There is no disclosure of compounds containing a drug or other compound of interest, including a leaving group, bound to a polymer.

Greenwald R.B., Expert Opinion on Therapeutic Patents (1997) Vol. 7, No. 6, 601-609, reviews various anticancer prodrugs and their polymeric conjugates as drug delivery systems.

EP 648503 describes various substrate-spacer-prodrug compounds for site specific delivery of drugs. Prodrugs are defined herein as drug compounds attached via a spacer to glycosyl, wherein glycosyl means a mono-, oligo- or polysaccharide or derivative thereof.

It is believed that in spite of the reported work in the field of double prodrugs, some specific problems were not addressed sufficiently. For example, the previously reported techniques do not sufficiently address the solubility problems of many parent compounds. In addition, the problem of designing in a sufficient increase in circulating half-life for the prodrug was also not sufficiently developed. Thus, there continues to be a need to provide additional technologies for forming prodrugs which would benefit from the double prodrug concept. For example, it would be advantageous to provide the artisan with alternative techniques for transport carrier attachment so as to regulate biological effect. Furthermore, it would be desirable to provide additional techniques to address problems associated with involving amino residues of parent compounds and thus avoid excessively fast or slow hydrolysis of the transport form from the parent compound at physiological pH.

### SUMMARY OF THE INVENTION

The present invention addresses the shortcomings described above. The compounds that are provided according to the invention, are summarised in the appended claims. In one aspect of the disclosure compounds of Formula (I) are provided: 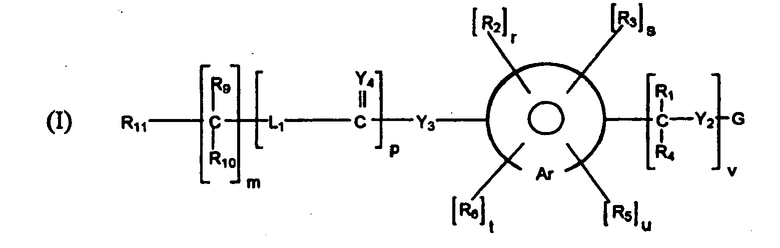 wherein:
L₁ is a bifunctional linking moiety such as 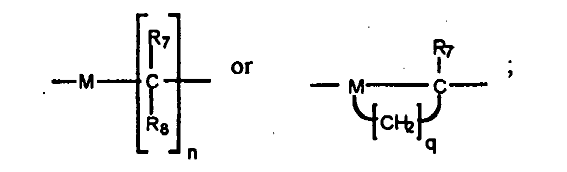 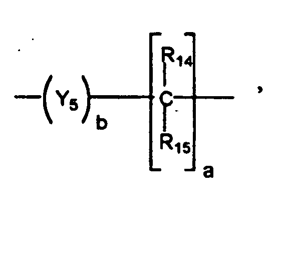
G is 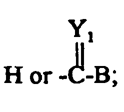 where
B is H, a leaving group, a residue of an amine-containing moiety, or a residue of a hydroxyl-containing moiety;
Y₁₋₅ are independently O, S or NR₁₂;
M is X or Q; where
X is an electron withdrawing group
Q is a moiety containing a free electron pair positioned three to six atoms from 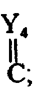
R₁, R₄, R₇, R₈, R₉, R₁₀, R₁₂, R₁₄ and R₁₅ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls;
R₂, R₃, R₅ and R₆ are independently selected from the group consisting of hydrogen, C₁₋₆ alkyls, C₁₋₆ alkoxy, phenoxy, C₁₋₈ heteroalkyls, C₁₋₈ heteroalkoxy, substituted C₁₋₆ alkyls, C₃₋₈ cycloalkyls, C₃₋₈ substituted cycloalkyls, aryls, substituted aryls, aralkyls, halo-, nitro- and cyano-, carboxy-, carboxyalkyl, alkylcarbonyl, etc.;
Ar is a moiety which when included in Formula (I) forms a multi-substituted aromatic hydrocarbon or a multi-substituted heterocyclic group;
(b), (m), (r), (s), (t), (u), and (v) are independently zero or one;
(a) and (n) are independently zero or a positive integer;
(p) is zero or a positive integer;
(q) is three or four; and
R₁₁ is a polymer such as a polyalkylene oxide.

In some preferred embodiments, (r) and (t) are one and R₂ and R₆ are independently selected from C₁₋₆ alkoxy or C₁₋₆ alkyl moieties and R₃ and R₅ are both hydrogen.

In other preferred embodiments, (v) is zero, 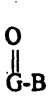 where B is hydrogen. This aldehyde derivative of Formula (I) provides useful intermediates for forming prodrug compositions.

or component a leaving group such as N-hydroxy-benzotriazolyl, N-hydroxyphthalimidyl, *p*-nitrophenoxy, imidazolyl, N-hydroxysuccinimidyl, thiazolidyl thione, or other activating groups, B is a residue of an amino-containing or hydroxyl-containing compound such as for which one or more of improved aqueous solubility, decreased antigenicity, prodrug and/or controlled release delivery is desired. For example, B, can be a residue of an enzyme, protein, or organic compound such as daunorubicin, doxorubicin, *p*-aminoaniline mustard, camptothecin, paclitaxel, Ara-C, melphalan, podophyllotoxin, etc.

For purposes of the present invention, the term "residue" shall be understood to mean that portion of a biologically active compound which remains after it has undergone a substitution reaction in which the prodrug carrier portion has been attached.

For purposes of the present invention, the term "alkyl" shall be understood to include straight, branched, substituted C₁₋₁₂ alkyls, C₃₋₈ cycloalkyls or substituted cycloalkyls, etc.

The double prodrugs of the present invention are thus unique delivery systems. Preferably the polymeric portion is first released by hydrolysis and then the resultant "second prodrug" moiety undergoes a 1,4 or 1,6-aryl or benzyl elimination reaction to regenerate the amine-containing bioactive compound.

Some of the chief advantages of the double prodrug compounds of the present invention are that they are capable of solubilizing amine-containing or hydroxyl-containing compounds and extending their half-life as compared to the native or even "second" prodrug counterparts. The linkage between the polymer and the "second prodrug" compound as described above, hydrolyzes at a rate which allows the compound to retain its enhanced solubility and circulating half-life. The native drug, however, is still not released at this point. Only after the "second prodrug" undergoes 1,4 or 1,6 -benzyl elimination, will the desired native molecule be released. It is readily apparent that this double prodrug approach of the present invention offers unique and unexpected characteristics which enhance the circulating half-life and solubility of native molecules.

Methods of making and using the compounds and conjugates described herein are also provided.

### BRIEF DESCRIPTION OF THE DRAWINGS

Figure 1 depicts the three synthetic methods for making the polymeric double prodrugs of the present disclosure
Figures 2 through 6 are illustrative reaction schemes corresponding to compounds prepared in the examples, among which compounds 3a, 3b, 4a, 4b, 5b, 6b, 7b, 8a, 8b, 9b, 10b, 13a, 13b, 14a, 14b, 15-18a, 18b, 24a, 24b, 26b, 27b, 30b, 32b, 32d, 36-40 and 47-50 are not compounds according to the invention.

### DETAILED DESCRIPTION OF THE INVENTION

### A. FORMULA (I)

In one aspect of the disclosure there are provided compounds of formula (I): 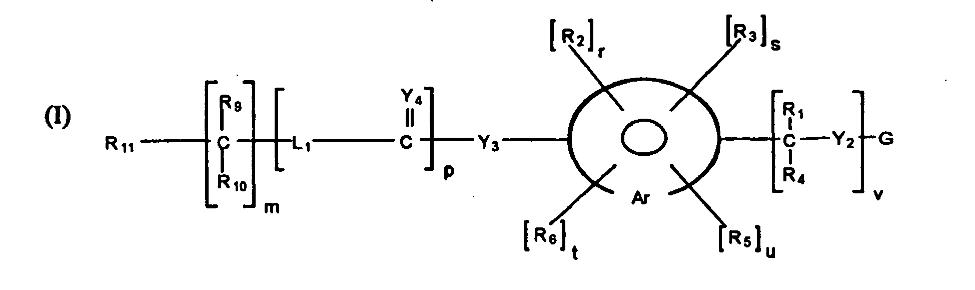 wherein:
L₁ is a bifunctional linking moiety such as 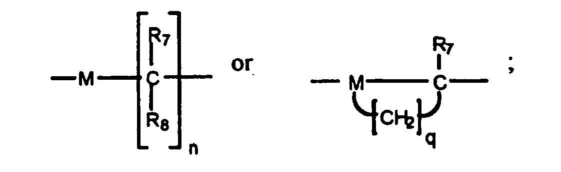 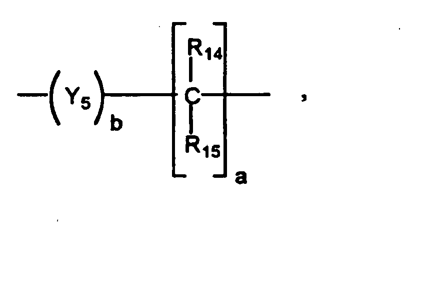
GisHor 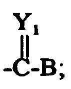 where
B is H, a leaving group, a residue of an amine-containing moiety, or a residue of a hydroxyl-containing moiety;
Y₁₋₅ are independently O, S or NR₁₂;
M is X or Q; where
X is an electron withdrawing group;
Q is a moiety containing a free electron pair positioned three to six atoms from 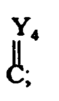
R_{1,} R₄, R₇, R₈, R₉, R₁₀, R₁₂, R₁₄ and R₁₅ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls;
R₂, R₃, R₅ and R₆ are independently selected from the group consisting of hydrogen, C₁₋₆ alkyls, C₁₋₆ alkoxy, phenoxy, C₁₋₈ heteroalkyls, C₁₋₈ heteroalkoxy, substituted C₁₋₆ alkyls, C₃₋₈ cycloalkyls, C₃₋₈ substituted cycloalkyls, aryls, substituted aryls, aralkyls, halo-, nitro- and cyano-, carboxy-, carboxyalkyl, alkylcarbonyl, etc.;
Ar is a moiety, which when included in Formula (I), forms a multi-substituted aromatic hydrocarbon or a multi-substituted heterocyclic group;
(b), (m), (r), (s), (t), (u) and (v) are independently zero or one;
(a) and (n) are independently zero or a positive integer, preferably 1-6 inclusive;
(p) is zero or a positive integer, preferably 1-6 inclusive;
(q) is three or four; and
R₁₁ is a substantially non-antigenic polymer.

### B. DESCRIPTION OF THE Ar MOIETY

Referring to Formula (I), it can be seen that the *Ar* moiety is a moiety which when included in Formula (I) forms a multi-substituted aromatic hydrocarbon or a multi-substituted heterocyclic group. A key feature is that the *Ar* moiety is aromatic in nature. Generally, to be aromatic, the π electrons must be shared within a "cloud" both above and below the plane of a cyclic molecule. Furthermore, the number of π electrons must satisfy the Hückle rule (4n+2). Those of ordinary skill will realize that a myriad of moieties will satisfy the aromatic requirement of the moiety and thus are suitable for use herein.

Preferred aromatic hydrocarbon moieties include, without limitation: 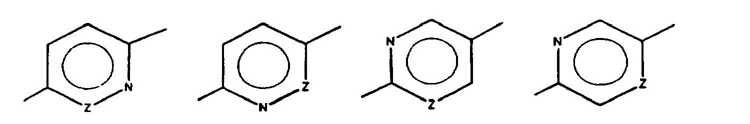 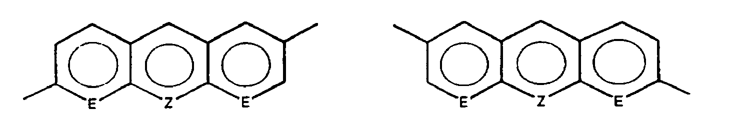 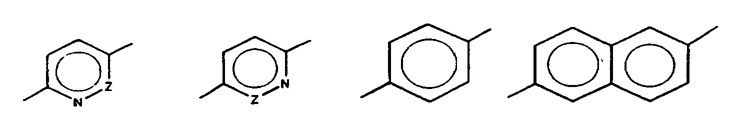 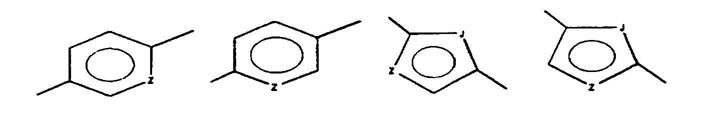 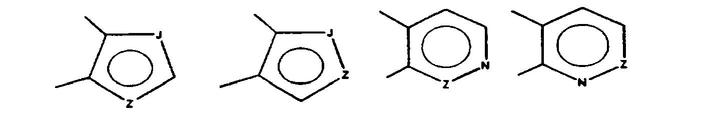 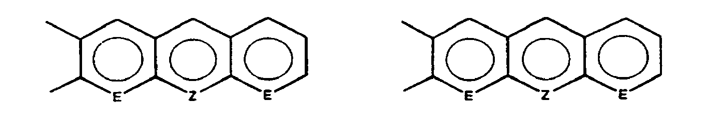 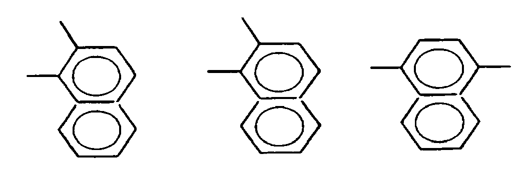 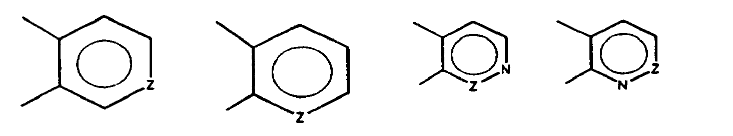 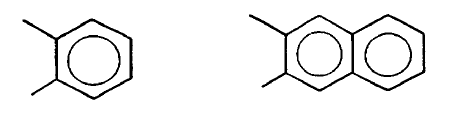 wherein J is O, S, or NR₁₃, E and Z are independently CR₁₃ or NR₁₃; and R₁₃ is independently selected from the same group as that which defines R₉ in Formula (I) e.g. hydrogen, C₁₋₆ alkyls, etc. Isomers of the five and six-membered rings are also contemplated as well as benzo- and dibenzo- systems and their related congeners are also contemplated. It will also be appreciated by the artisan of ordinary skill that the aromatic rings can optionally be substituted with heteroatoms such as O, S, NR₁₃, etc. so long as Hückel's rule is obeyed. Furthermore, the aromatic or heterocyclic structures may optionally be substituted with halogen(s) and/or side chains as those terms are commonly understood in the art. However, all structures suitable for Ar moieties of the present disclosure are capable of allowing the Y₃ and C(R₁)(R₄) moieties to be in a *para* or an *ortho* arrangement with the same plane as shown: 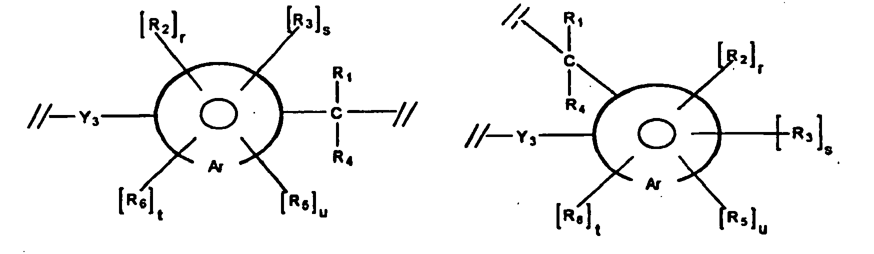 where all variables are as defined above with regard to Formula (I).

When the Ar moiety includes a *para* arrangement of the Y₃ and C(R₁)(R₄) moieties, preferred aspects of the present invention define (r), (s), (t), and (u) as one and R₂ and R₆ as being independently selected from the group consisting of methyl, C₁₋₆ alkyls, methyl, C₁₋₆ alkoxys, and methoxy. More preferably, R₂ and R₆ are either both methyl or methoxy moieties. Furthermore, R₃ and R₅ are preferably both hydrogen, R₁ and R₄ are preferably either hydrogen, CH₃ or CH₂CH₃. Y₁₋₅ are preferably O or NR₁₂ where R₁₂ is H or a C₁₋₆ alkyl or substituted alkyl. More preferably, Y₁ and Y₄ are O.

For purposes of the present disclosure substituted alkyls include carboxyalkyls, aminoalkyls, dialkylaminos, hydroxyalkyls and mercaptoalkyls; substituted cycloalkyls include moieties such as 4-chlorocyclohexyl; aryls include moieties such as napthyl; substituted aryls include moieties such as 3-bromophenyl; aralkyls include moieties such as toluyl; heteroalkyls include moieties such as ethylthiophene; substituted heteroalkyls include moieties such as 3-methoxythiophene; alkoxy includes moieties such as methoxy; and phenoxy includes moieties such as 3-nitrophenoxy. Halo- shall be understood to include fluoro, chloro, iodo and bromo.

### C. LINKER MOIETY L₁

As shown above, the disclosure includes bifunctional linking moiety L₁ which when combined with 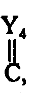 forms an amino acid residue linker, or when (p) is greater than one, a peptide residue linker.

Suitable amino acid residues can be selected from naturally-occurring or synthetic, i.e. non-naturally-occurring, amino acids including alanine, valine, leucine, isoleucine, glycine, serine, threonine, methionine, cysteine, phenylalanine, tyrosine, tryptophan, aspartic acid, glutamic acid, lysine, arginine, histidine or proline. Some preferred peptide residues include Gly-Phe-Leu-Gly and Gly-Phe-Leu. It is noted that the terminal amino group of the amino acid or peptide residue will be proximal to R₁₁ (i.e. polymer). Peptides can be readily synthesized or obtained from commercial sources for inclusion herein.

In alternative embodiments, L₁ includes the moiety (M) which is either an electron withdrawing group (designated herein as X), or a moiety containing a free electron pair positioned three to six atoms from the 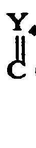 (designated herein as Q).

### D. THE DOUBLE PRODRUG LINKAGE PORTION

The first labile bond of the double prodrug system, which joins the L₁ to 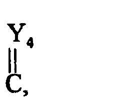 is selected to hydrolyze, such as via an esterase catalyzed hydrolysis *in vivo* at a rate which generates sufficient amounts of the "second" prodrug compound within a suitable time after administration. The term "sufficient amounts" for purposes of the present invention shall mean an amount which may later undergo sufficient 1,4 or 1,6 -benzyl elimination *in vivo* to release the native compound and achieve a desired effect. Preferably, *(n)* is an integer from 1 to about 12. More preferably, *(n)* is 1 or 2.

### 1. The Electron Withdrawing Group X

In those aspects of Formula (I) where L₁ includes M, the moiety may be an electron withdrawing group, designated herein as X. For purposes of the present invention, "electron withdrawing groups" are groups which tend to pull shared electrons toward themselves thereby making carbon more electro-positive. This, in turn, destabilizes the carbonyl moiety, causing more rapid hydrolysis. Thus, when X is in the α position to the ester, it modulates the rates of hydrolysis and enzymatic cleavage. In particular, X can be moieties such as O, NR₁₂, 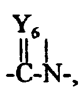 S, SO and SO₂ where Y₆ is the same as that defined by Y₁ and R₁₂ is as defined above, i.e. H, C₁₋₆ alkyls, branched alkyls, aryls, etc. Preferably, however, when X is NR₁₂, R₁₂ is H, a C₁₋₆ alkyl such as methyl or ethyl or substituted C₁₋₆ alkyl. It is preferred that X is either O or NR₁₂.

### 2. Q Portion of the Linker

Alternatively, when L₁ includes Q, which is a moiety containing a free electron pair positioned three to six atoms from the 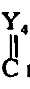 moiety, the polymer, R₁₁, is preferably attached to Q via a heteroatom such as oxygen. In a preferred embodiment, the free electron pair is five atoms from this oxygen. Q can be selected from the non-limiting list of C₂₋₄ alkyls or cycloalkyls, aryls or aralkyl groups substituted with a member of the group consisting of O, S and NR₁₂. The free electron pair can be anywhere along the Q moiety as long as the defined spacing between the free electron pair and Y₄ is maintained.

In these embodiments, R₁₁ is attached to Q via NR₁₂, O, or S. Thus, Q assists hydrolysis of the prodrug linkage by anchimeric assistance because the free electron pair moiety can generate a three- to six-membered, but preferably five-membered, ring by-product upon hydrolysis of the preferably ester linkage.

Q can also be selected from the group consisting of C₂₋₄ alkyls, cycloalkyls, aryls, aralkyl groups substituted with a member of the group consisting of NH, O, S, -CH₂-C(O)-N(H)-, and ortho-substituted phenyls such as

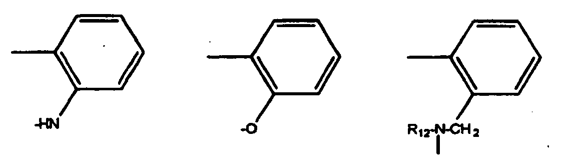

### 3. Drug Generation Via Hydrolysis of the Prodrugs

The prodrug compounds of the present invention are designed so that the t_{1/2} of hydrolysis is < t_{1/2} elimination in plasma.

The linkages included in the compounds have hydrolysis rates in the plasma of the mammal being treated which is short enough to allow sufficient amounts of the parent compounds, i.e. the amino- or hydroxyl-containing bioactive compound, to be released prior to elimination. Some preferred compounds of the present disclosure i.e. those in which (*n*) is 1, have a t_{1/2} for hydrolysis in plasma ranging from about 5 minutes to about 12 hours. Preferably, the compositions have a plasma t_{1/2} hydrolysis ranging from about 0.5 to about 8 hours and most preferably from about 1 to about 6 hours.

### 4. 1,4 or 1,6 -Benzyl Elimination and Native Drug Regeneration

Once the hydrolysis of the double prodrug has taken the place *in vivo,* usually via esterase activity or pH moderated activity or cyclization reaction, the polymeric residue is cleaved and the resultant second prodrug moiety remains. According to the present invention, this prodrug entity will undergo a further step of a 1,4 or 1,6-benzyl elimination *in vivo* to produce the desired native compound by electron migration causing the following irreversible decomposition which regenerates the drug. For example, when the Y₃ and C(R₁)(R₄) moieties of the double prodrugs of the present disclosure form a *para* arrangement, a representative reaction is shown below with Y₂, Y₃, and Y₄ being O; R₁ and R₄ being H; and G being C(O)-B where B is a residue of an amine-containing target moiety (i.e. NH₂-Drug).

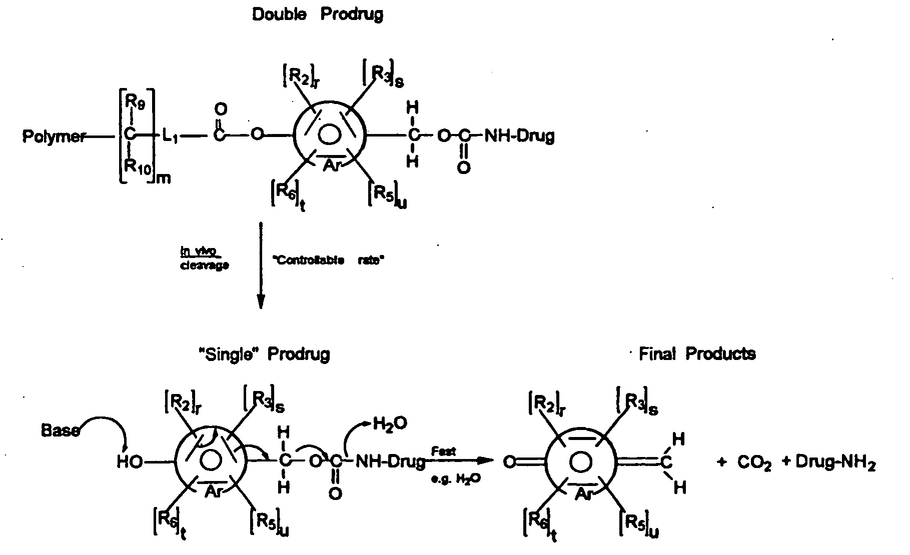

Although not shown, when the Y₃ and C(R₁)(R₄) of the double prodrugs of the present invention are in an *ortho* arrangement, the reaction proceeds in a similar manner.

### E. SUBSTANTIALLY NON-ANTIGENIC POLYMERS

The "double prodrug" compositions of the present invention include a water-soluble polymer.

Suitable examples of such polymers include polyethylene glycols (PEG) which are also preferably substantially non-antigenic.

PEG's can vary substantially in number average molecular weight, polymers ranging from about 2,000 to about 100,000 daltons are usually selected for the purposes of the present invention. Molecular weights of from about 5,000 to about 50,000 are preferred and 5,000 to about 40,000 are particularly preferred. The number average molecular weight of the polymer selected for inclusion in the "double prodrug" must be sufficient so as to provide sufficient circulation of the "double prodrug" before hydrolysis of the linker. Within the ranges provided above, polymers having molecular weight ranges of at least 20,000 are preferred in some aspects for chemotherapeutic and organic moieties. In the case of some nucleophiles such as certain proteins, enzymes and the like, polymers having a molecular weight range of from about 2,000 to about 20,000 are preferred.

The polymeric substances included herein are preferably water-soluble at room temperature.

For purposes of the present invention, "effectively non-antigenic" means all polymeric materials understood in the art as being nontoxic and not eliciting an appreciable immune response in mammals.

### F. POLYMERIC DOUBLE PRODRUG TRANSPORT SYSTEM SYNTHESIS

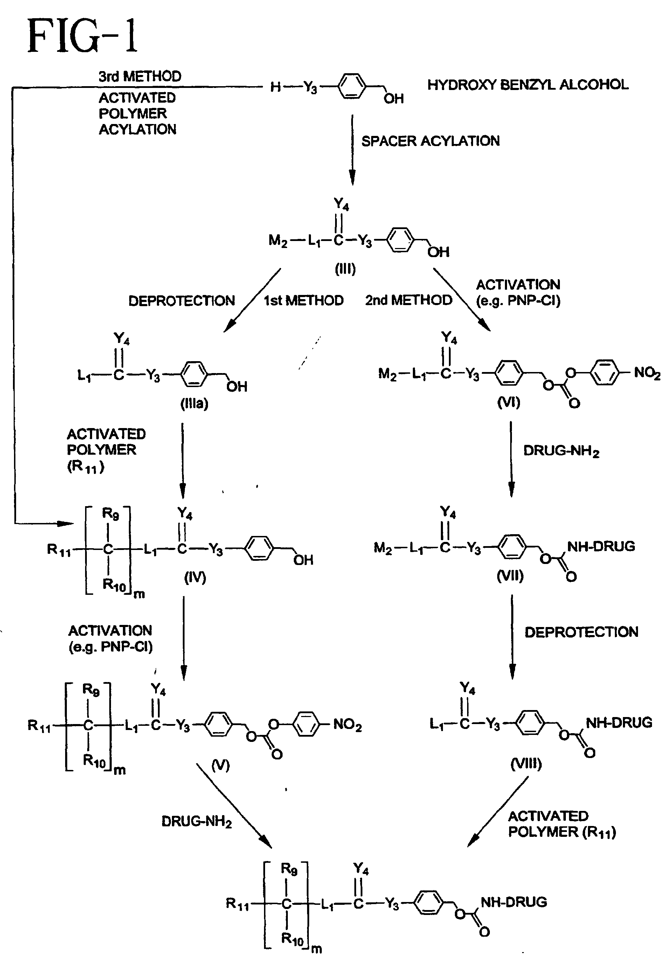

Synthesis of representative, specific prodrugs is set forth in the Examples. Generally, however, the double prodrugs of the present disclosure can be prepared in several fashions. See Figure 1. Thus, one method includes
a. providing an intermediate compound (III) 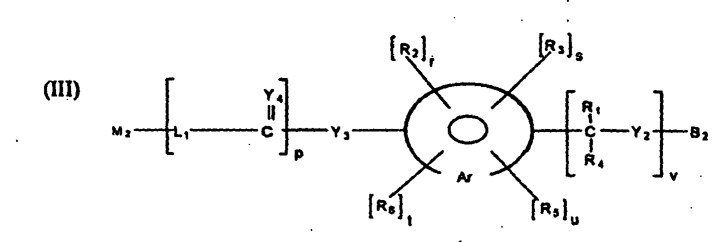 where M₂ is a cleavable or reversible protecting group, B₂ is H, OH, 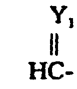 or a leaving group and all other variables are as set forth above with regard to Formula (I);
b. removing the protecting group such as by treating the intermediate compound (IIIa) with a strong acid such as TFA (trifluoroacetic acid) or other trihaloacetic acid, HCl, sulfuric acid, etc., or tetrabutyl ammonium fluoride;
c. reacting the resultant unprotected, intermediate compound (IIIa) with a moiety capable of reacting with a L₁ such as an activated polymer, i.e. a polymer having a reactive functional group, e.g., *p*-nitrophenyl or succinimidyl carbonate, carbonyl imidazole, thiazolidyl thione or the like, and optional spacer group, i.e. CR₉R₁₀, to form an intermediate activated double prodrug transport form of formula (IV): 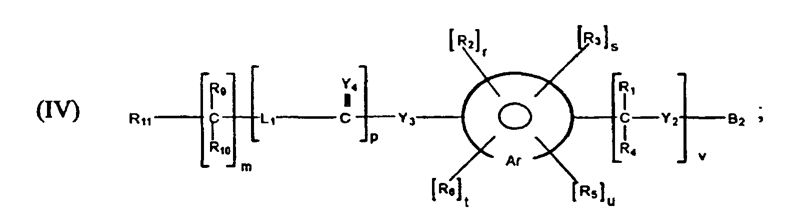
d. reacting the intermediate activated double prodrug transport form (IV) with an activating moiety donor such as *p*-nitrophenyl chloride (PNP-Cl) (forming, for example, compound (V) in Figure 1); and optionally
e. attaching an amine-containing or hydroxyl-containing compound residue, e.g. the drug to be transported, to compound (V) by displacing the leaving group in a substitution reaction with an amine-or hydroxyl-containing compound. Figure 1 illustrates the reaction with a benzyl derivative. Similar techniques are employed when other aromatic moieties are used as starting materials.

Alternatively, as also shown in Figure 1 with an amine-containing compound for illustrative purposes, the double prodrug can be prepared by:
a. providing an intermediate compound (III) as shown in the first method above and reacting it with an activating moiety donor such as *p*-nitrophenyl chloride (PNP-Cl) forming (VI) in Figure 1;
b. attaching an amine-containing or hydroxyl-containing compound, e.g. the drug to be transported, to the activated intermediate compound (VI);
c. removing the protecting group to form VII in Figure 1 (in the same manner as described above); and
d. reacting the unprotected intermediate (VIII in Figure 1) with an activated polymer to form the double prodrug.

Although not illustrated in Figure 1, the reaction scheme for a hydroxyl-containing compound would nonetheless proceed in a similar manner.

As shown in Figure 1, intermediate compound (III) can be prepared using standard organic synthesis techniques in which a hydroxy benzyl alcohol or other hydroxy aromatic alcohol is acylated with a spacer providing moiety.

In the third method illustrated in Figure 1, a hydroxy or amino aromatic alcohol such as hydroxy-benzyl or amino-benzyl alcohol is reacted with an activated polymer to form (IV) which is then converted to the final product following steps d) and e) of the first method described above.

Examples of suitable *o*-hydroxybenzyl alcohols include 6-hydroxybenzyl alcohol, 6-hydroxy-3,5-dimethylbenzyl alcohol, 6-hydroxy-3,5-dimethoxybenzyl alcohol, 6-hydroxy-3-methoxybenzyl alcohol.

Examples of suitable *p-*hydroxybenzyl alcohols include 4-hydroxybenzyl alcohol, 4-hydroxy-3,5-dimethylbenzyl alcohol, 4-hydroxy-3,5-dimethoxybenzyl alcohol, 4-hydroxy-3-methoxybenzyl alcohol.

Examples of suitable aminobenzyl alcohols include 2-aminobenzyl alcohol, 4-aminobenzyl alcohol, and 2-amino-3-methyl-or 3-alkyl benzyl alcohols.

Preferably, the final prodrugs are prepared in an inert solvent such as methylene chloride, chloroform, toluene, DMF or mixtures thereof The reaction also preferably is conducted in the presence of a base, such as dimethylaminopyridine, diisopropylethylamine, pyridine, triethylamine, etc. to neutralize any acids generated and at a temperature from -10°C up to about 45°C. The resulting conjugated prodrug composition is then recovered or isolated using techniques known to those of ordinary skill, i.e. filtered, recrystallized.

### G. THE LEAVING GROUP OR RESIDUE PORTION "B"

### 1. Leaving Groups

In those aspects where B is a leaving group, suitable leaving groups include, without limitations, moieties such as N-hydroxybenzotriazolyl, N-hydroxyphthalimidyl, *p*-nitrophenoxy, imidazolyl, N-hydroxysuccinimidyl; thiazolidinyl thione, or other good leaving groups as will be apparent to those of ordinary skill. The synthesis reactions used and described herein will be understood by those of ordinary skill without undue experimentation.

For example, the acylated intermediate compound (III) can be reacted with such as 4-nitrophenyl-chloroformate, disuccinimidyl carbonate (DSC), carbonyldiimidazole, thiazolidine thione, etc. to provide the desired activated derivative.

The acylation of the *p*-hydroxybenzyl alcohol or the *p-*aminobenzyl alcohol and the *o*-hydroxbenzyl alcohol or the *o-*aminobenzyl alcohol can be carried out with, for example, thiazolidine thione activated polymers, succinimidyl carbonate activated polymers, carboxylic acid activated polymers, blocked amino acid derivatives.

Once in place, the "activated" form of the PEG prodrug (or blocked prodrugs) is ready for conjugation with an amine- or hydroxyl-containing compound. Some preferred activated transport forms are shown below. 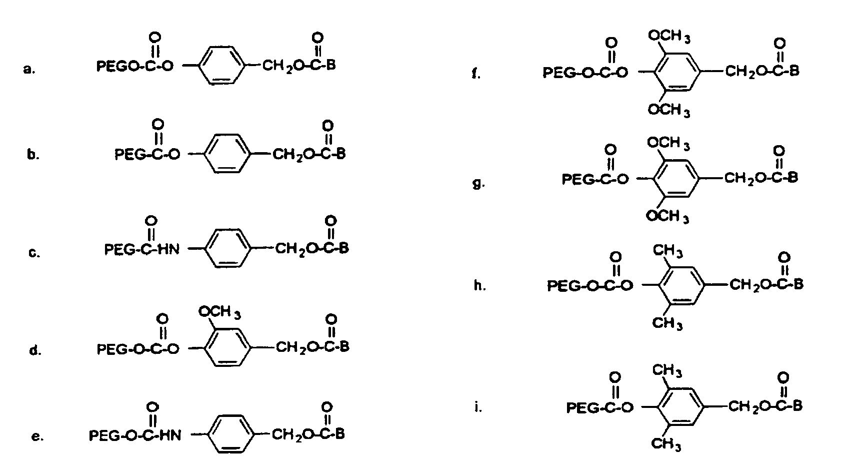 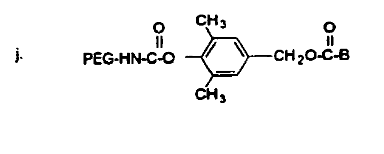 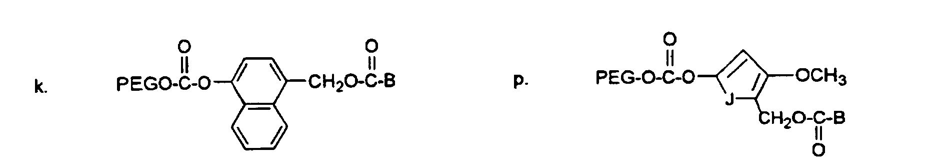 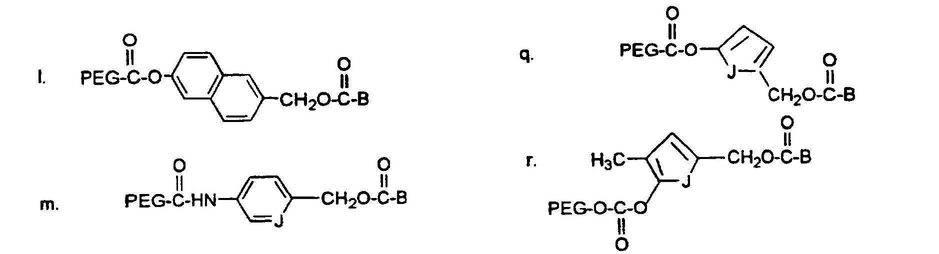 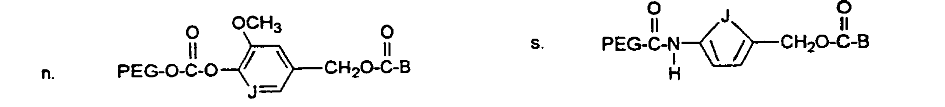 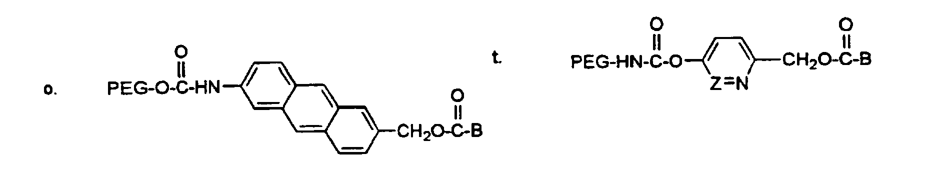 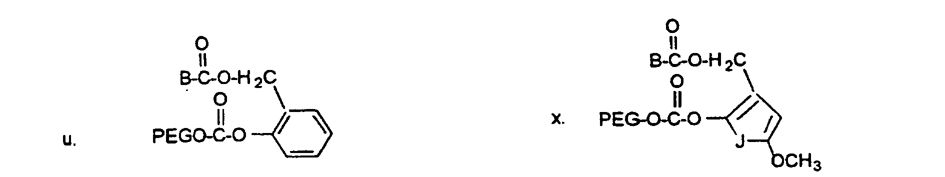 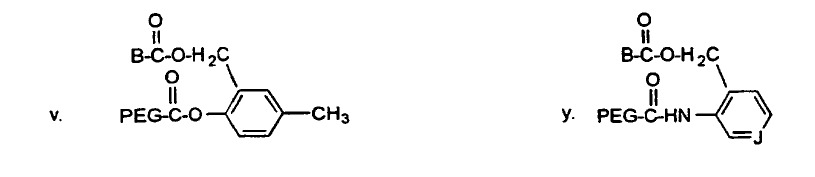 and

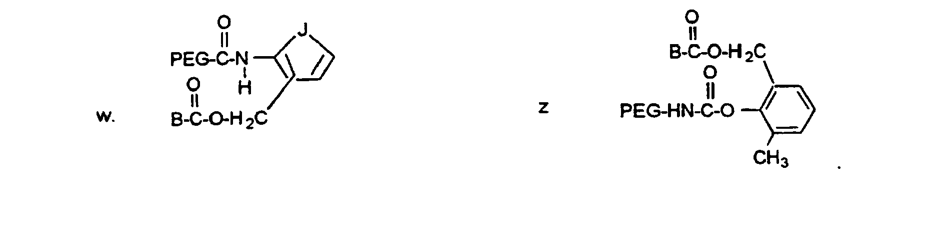

### 2. Residues of Amine-containing Compounds

In some aspects of the invention, e.g after the prodrug transport has been formed, B is a residue of an amine-containing compound, a non-limiting list of such suitable compounds include residues of organic compounds, enzymes, proteins, polypeptides, etc. Organic compounds include, without limitation, moieties such as anthracycline compounds including daunorubicin, doxorubicin; *p*-aminoaniline mustard, melphalan, Ara-C (cytosine arabinoside) and related anti-metabolite compounds, e.g., gemcitabine, etc. Alternatively, B can be a residue of an amine-containing cardiovascular agent, anti-neoplastic, anti-infective, anti-fungal such as nystatin and amphotericin B, anti-anxiety agent, gastrointestinal agent, central nervous system-activating agent, analgesic, fertility agent, contraceptive agent, anti-inflammatory agent, steroidal agent, anti-urecemic agent, vasodilating agent, vasoconstricting agent, etc.

Suitable proteins, polypeptides, enzymes, peptides and the like having at least one available amino group for polymer attachment include materials which have physiological or pharmacological activities as well as those which are able to catalyze reactions in organic solvents. The only other requirement of the amine-containing materials is that they maintain at least some portion of the activity associated with the unmodified protein, enzyme, peptide, etc. after the prodrug transport portion has hydrolyzed.

Proteins, polypeptides and peptides of interest include, but are not limited to, hemoglobin, serum proteins such as blood factors including Factors VII, VIII, and IX; immunoglobulins, cytokines such as interleukins, i.e. IL-1 through ML-13, α-, β- and γ- and consensus interferons, colony stimulating factors including granulocyte colony stimulating factors, platelet derived growth factors and phospholipase-activating protein (PLAP). Other proteins of general biological or therapeutic interest include insulin, plant proteins such as lectins and ricins, tumor necrosis factors and related proteins, growth factors such as transforming growth factors, such as TGFα's or TGFβ's and epidermal growth factors, hormones, somatomedins, erythropoietin, pigmentary hormones, hypothalamic releasing factors, antidiuretic hormones, prolactin, chorionic gonadotropin, follicle-stimulating hormone, thyroid-stimulating hormone, tissue plasminogen activator, and the like. Immunoglobulins of interest include IgG, IgE, IgM, IgA, IgD and fragments thereof.

Some proteins such as the interleukins, interferons and colony stimulating factors also exist in non-glycosylated form, usually as a result of using recombinant techniques. The non-glycosylated versions are also among the proteins of the present invention.

Enzymes of interest include carbohydrate-specific enzymes, proteolytic enzymes, oxidoreductases, transferases, hydrolases, lyases, isomerases and ligases. Without being limited to particular enzymes, examples of enzymes of interest include asparaginase, arginase, arginine deaminase, adenosine deaminase, superoxide dismutase, endotoxinases, catalases, chymotrypsin, lipases, uricases, adenosine diphosphatase, tyrosinases and bilirubin oxidase. Carbohydrate-specific enzymes of interest include glucose oxidases, glucodases, galactosidases, glucocerebrosidases, glucouronidases, etc.

Also included herein is any portion of a polypeptide demonstrating *in vivo* bioactivity. This includes amino acid sequences, nucleic acids (DNA, RNA) peptide nucleic acids (PNA), antibody fragments, single chain binding proteins, see, for example U.S. Patent No. 4,946,778, disclosure of which is incorporated herein by reference, binding molecules including fusions of antibodies or fragments, polyclonal antibodies, monoclonal antibodies and catalytic antibodies.

The proteins or portions thereof can be prepared or isolated by using techniques known to those of ordinary skill in the art such as tissue culture, extraction from animal sources, or by recombinant DNA methodologies. Transgenic sources of the proteins, polypeptides, amino acid sequences and the like are also contemplated. Such materials are obtained from transgenic animals, i.e., mice, pigs, cows, etc., wherein the proteins are expressed in milk, blood or tissues. Transgenic insects and baculovirus expression systems are also contemplated as sources. Moreover, mutant versions of proteins, such as mutant interferons are also within the scope of the invention.

Other proteins of interest are allergen proteins such as ragweed, Antigen E, honeybee venom, mite allergen, and the like. The foregoing is illustrative of the proteins which are suitable for the present invention. It is to be understood that those proteins, as defined herein, not specifically mentioned but having an available amino group are also intended and are within the scope of the present invention.

In a preferred aspect of the invention, the amino-containing compound is a biologically active compound that is suitable for medicinal or diagnostic use in the treatment of animals, e.g., mammals, including humans, for conditions for which such treatment is desired. The foregoing list is meant to be illustrative and not limiting for the compounds which can be modified. Those of ordinary skill will realize that other such compounds can be similarly modified without undue experimentation. It is to be understood that those biologically active materials not specifically mentioned but having suitable amino-groups are also intended and are within the scope of the present invention.

The only limitations on the types of amino-containing molecules suitable for inclusion herein is that there is available at least one (primary or secondary) amine containing position which can react and link with a carrier portion and that there is not substantial loss of bioactivity after the double prodrug system releases and regenerates the parent compound.

It is noted that parent compounds suitable for incorporation into the double prodrug compositions of the invention, may themselves be substances/compounds which are not active after hydrolytic release from the linked composition, but which will become active after undergoing a further chemical process/reaction. For example, an anticancer drug that is delivered to the bloodstream by the double prodrug transport system, may remain inactive until entering a cancer or tumor cell, whereupon it is activated by the cancer or tumor cell chemistry, e.g., by an enzymatic reaction unique to that cell.

After conjugation, the remaining amine-containing compound is referred to as the residue of the unconjugated compound.

### 3. Residues of Hydroxyl-Containing Compounds

### a. Camptothecin and Related Topoisomerase I Inhibitors

Camptothecin is a water-insoluble cytotoxic alkaloid produced by *Camptotheca accuminata* trees indigenous to China and *nothapodytes foetida* trees indigenous to India. Camptothecin and related compounds and analogs are also known to be potential anticancer or antitumor agents and have been shown to exhibit these activities *in vitro* and *in vivo.* Camptothecin and related compounds are also candidates for conversion to the double prodrugs of the present invention. Camptothecin and certain related analogues share the structure:

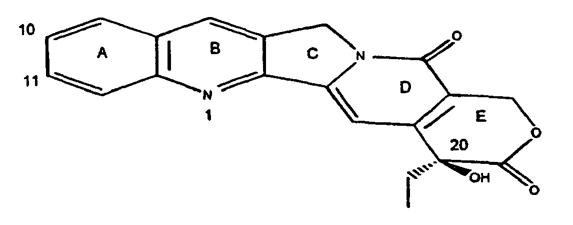

From this core structure, several known analogs have been prepared. For example, the A ring in either or both of the 10- and 11-positions can be substituted with an OH. The A ring can also be substituted in the 9-position with a straight or branched C₁₋₃₀ alkyl or C₁₋₁₇ alkoxy, optionally linked to the ring by a heteroatom i.e.- O or S. The B ring can be substituted in the 7-position with a straight or branched C₁₋₃₀ alkyl or substituted alkyl-, C₅₋₈ cycloakyl, C₁₋₃₀ alkoxy, phenyl alkyl, etc., alkyl carbamate, alkyl carbazides, phenyl hydrazine derivatives, amino-, aminoalkyl-, aralkyl, etc. Other substitutions are possible in the C, D and E rings. See, for example, U.S. Patent Nos. 5,004,758; 4,943,579; Re 32,518, the contents of which are incorporated herein by reference. Such derivatives can be made using known synthetic techniques without undue experimentation. Preferred camptothecin derivatives for use herein include those which include a 20-OH or another OH moiety which is capable of reacting directly with activated forms of the polymer transport systems described herein or to the linking moiety intermediates, e.g. iminodiacetic acid, etc., which are then attached to a polymer such as PEG. Reference to camptothecin analogs herein has been made for purposes of illustration and not limitation.

### b. Taxanes and Paclitaxel Derivatives

One class of compounds included in the double prodrug compositions of the present invention is taxanes. For purposes of the present invention, the term "taxane" includes all compounds within the taxane family of terpenes. Thus, taxol (paclitaxel), 3'-substituted tert-butoxy-carbonyl-amine derivatives (taxoteres) and the like as well as other analogs which are readily synthesized using standard organic techniques or are available from commercial sources such as Sigma Chemical of St. Louis, Missouri are within the scope of the present invention. Representative taxanes are shown below.

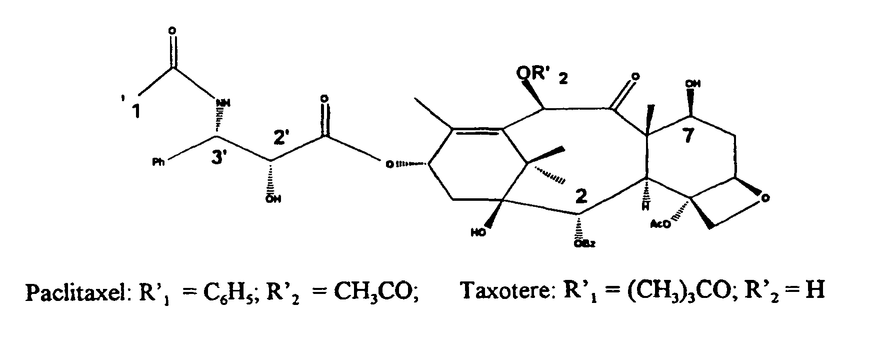

These derivatives have been found to be effective anti-cancer agents. Numerous studies indicate that the agents have activity against several malignancies. To date, their use has been severely limited by, among other things, their short supply, poor water solubility and hypersensitivity. It is to be understood that other taxanes including the 7-aryl-carbamates and 7-carbazates disclosed in commonly assigned U.S. Patent Nos. 5,622,986 and 5,547,981 can also be included in the double prodrugs of the present invention. The contents of the foregoing U.S. patents are incorporated herein by reference. The only limitation on the taxane is that it must be capable of undergoing a hydroxyl based substitution reaction such as at the 2' position. Paclitaxel, however, is a preferred taxane.

### c. Additional Biologically-Active Moieties

In addition to the foregoing molecules, the double prodrug formulations of the present invention can be prepared using many other compounds. For example, biologically-active compounds such as gemcitabine: 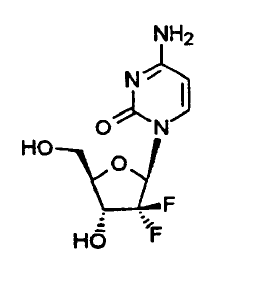 or etoposide: 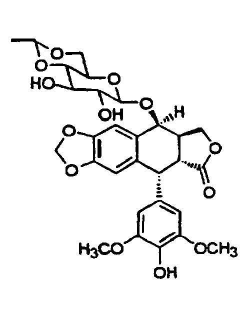 or triazole-based antifungal agents such as fluconazole: 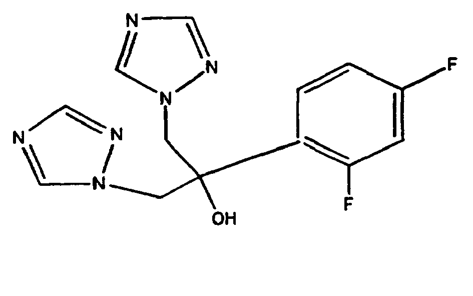 or ciclopirox: 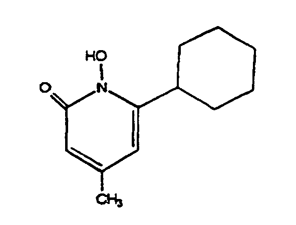 can be used.

The parent compounds selected for double prodrug forms need not be substantially water-insoluble, although the polymer-based double prodrugs of the present invention are especially well suited for delivering such water-insoluble compounds. Other useful parent compounds include, for example, certain low molecular weight biologically active proteins, enzymes and peptides, including peptido glycans, as well as other anti-tumor agents; cardiovascular agents such as forskolin; anti-neoplastics such as combretastatin, vinblastine, doxorubicin, Ara-C, maytansine, etc.; anti-infectives such as vancomycin, erythromycin, etc.; antifungals such as nystatin, amphoteracin B, triazoles, papulocandins, pneumocandins, echinocandins, polyoxins, nikkomycins, pradimicins, benanomicins, etc. see, "Antibiotics That Inhibit Fungal Cell Wall Development" Annu. Rev. Microbiol. 1994, 48:471-97, the contents of which are incorporated herein by reference; anti-anxiety agents, gastrointestinal agents, central nervous system-activating agents, analgesics, fertility or contraceptive agents, anti-inflammatory agents, steroidal agents, anti-urecemic agents, cardiovascular agents, vasodilating agents, vasoconstricting agents and the like.

It is noted that parent compounds suitable for incorporation into the double prodrug compositions of the invention, may themselves be substances/compounds which are not active after hydrolytic release from the linked composition, but which will become active after undergoing a further chemical process/reaction. For example, an anticancer drug that is delivered to the bloodstream by the double prodrug transport system, may remain inactive until entering a cancer or tumor cell, whereupon it is activated by the cancer or tumor cell chemistry, e.g., by an enzymatic reaction unique to that cell.

After conjugation, the remaining amine-or hydroxyl-containing compound is referred to as the residue of the unconjugated compound.

### 4. Polymeric Hybrids

In another aspect of the invention there are provided hybrid types of the polymeric double prodrug transport system described herein. In particular, the hybrid system includes not only the reversible double prodrug system described above but also a second polymeric transport system based on more permanent types of linkages. The hybrids can be prepared by at least two methods. For example, the benzyl-elimination-based double prodrug can be synthesized first and then PEGylated using any art-recognized activated polymer such as thiazolidinyl thione- or succinimidyl carbonate-activated PEG. Alternatively, the more permanent conjugation reaction can be performed first and the resultant conjugates can be used to form the double prodrug conjugates described herein. It will be understood that the hybrid systems will be better suited for proteins, enzymes and the like where multiple amino groups are available for attachment of the polymeric transport forms. For purposes of the present invention, "activated polymers" will be understood to include polymers containing one or more terminal groups which are capable of reacting with one or more of α-amino groups, ∈-amino groups, histidine nitrogens, carboxyl groups, sulfhydryl groups, etc. found on enzymes, proteins, etc., as well as such groups found on synthetically prepared organic compounds. It will further be appreciated that the activating groups described below can also be used to from the activated transport forms described above.

The activating terminal moiety can be any group which facilitates conjugation of the polymers with the biologically active material, i.e. protein, enzyme, etc. either before of after the double prodrug transport system of the present invention has been synthesized. See, for example, U.S. Patent No. 4,179,337, the disclosure of which is hereby incorporated by reference. Such activating groups can be a moiety selected from:
I. Functional groups capable of reacting with an amino group such as:
   a) carbonates such as the p-nitrophenyl, or succinimidyl; see, for example, U.S. Patent No. 5,122,614, the disclosure of which is hereby incorporated by reference;
   b) carbonyl imidazole;
   c) azlactones; see, for example, U.S. Patent No. 5,321,095, the disclosure of which is hereby incorporated by reference;
   d) cyclic imide thiones see, for example, U.S. Patent No. 5,349,001, the disclosure of which is hereby incorporated by reference;
   e) isocyanates or isothiocyanates; or
   f) active esters such as N-hydroxy-succinimidyl or N-hydroxybenzotriazolyl.
II. Functional groups capable of reacting with carboxylic acid groups and reactive carbonyl groups such as:
   a) primary amines; or
   b) hydrazine and hydrazide functional groups such as the acyl hydrazides, carbazates, semicarbamates, thiocarbazates, etc.
III. Functional groups capable of reacting with mercapto or sulfhydryl groups such as phenyl glyoxals; see, for example, U.S. Patent No. 5,093,531, the disclosure of which is hereby incorporated by reference;
IV. Functional groups capable of reacting with hydroxyl groups such as (carboxylic) acids or other nucleophiles capable of reacting with an electrophilic center. A non-limiting list includes, for example, hydroxyl, amino, carboxyl, thiol groups, active methylene and the like.

The activating moiety can also include a spacer moiety located proximal to the polymer. The spacer moiety may be a heteroalkyl, alkoxy, alkyl containing up to 18 carbon atoms or even an additional polymer chain. The spacer moieties can added using standard synthesis techniques.

### H. METHODS OF TREATMENT

Compounds of the present invention may be used in methods of treatment for various medical conditions in mammals. The methods include administering to the mammal in need of such treatment, an effective amount of a composition of the invention, as described herein, such as a double prodrug of doxorubicin The prodrug compositions are useful for, among other things, treating diseases which are similar to those which are treated with the parent compound, e.g. enzyme replacement therapy, neoplastic disease, reducing tumor burden, preventing metastasis of neoplasms and preventing recurrences of tumor/neoplastic growths in mammals.

The amount of the prodrug that is administered will depend upon the amount of the parent molecule included therein. Generally, the amount of prodrug used in the treatment methods is that amount which effectively achieves the desired therapeutic result in mammals. Naturally, the dosages of the various prodrug compounds will vary somewhat depending upon the parent compound, rate of in *vivo* hydrolysis, molecular weight of the polymer, etc. In general, double prodrug polymeric derivatives are administered in amounts ranging from about 5 to about 500 mg/m² per day, based on the native drug. The range set forth above is illustrative and those skilled in the art will determine the optimal dosing of the prodrug selected based on clinical experience and the treatment indication. Actual dosages will be apparent to the artisan without undue experimentation.

The compositions, including prodrugs, of the present invention can be included in one or more suitable pharmaceutical compositions for administration to mammals. The pharmaceutical compositions may be in the form of a solution, suspension, tablet, capsule or the like, prepared according to methods well known in the art. It is also contemplated that administration of such compositions may be by the oral and/or parenteral routes depending upon the needs of the artisan. A solution and/or suspension of the composition may be utilized, for example, as a carrier vehicle for injection or infiltration of the composition by any art known methods, e.g., by intravenous, intramuscular, subdermal injection and the like.

Such administration may also be by infusion into a body space or cavity, as well as by inhalation and/or intranasal routes. In preferred aspects of the invention, however, the prodrugs are parenterally administered to mammals in need thereof.

### I. EXAMPLES

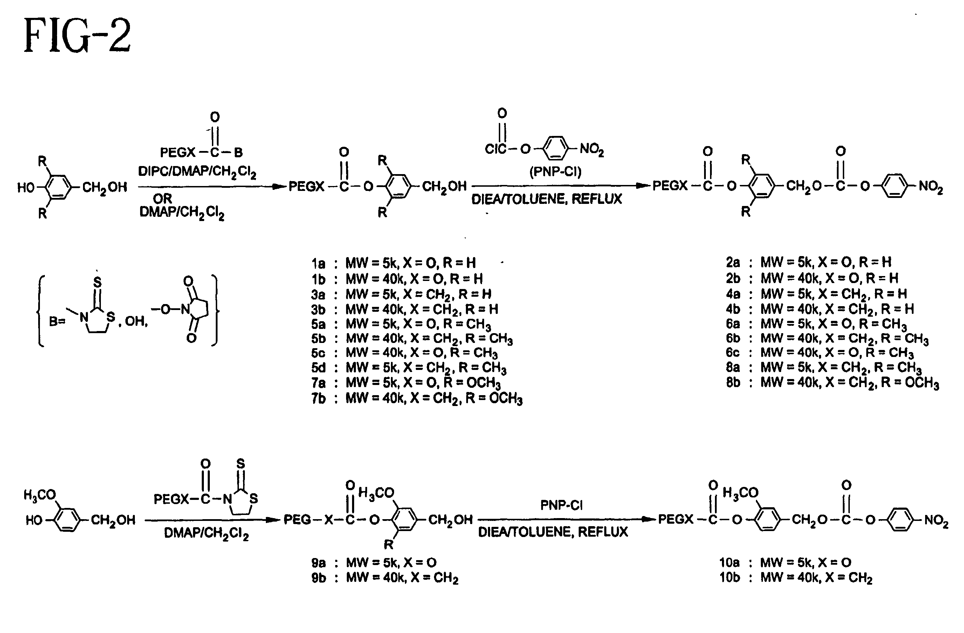

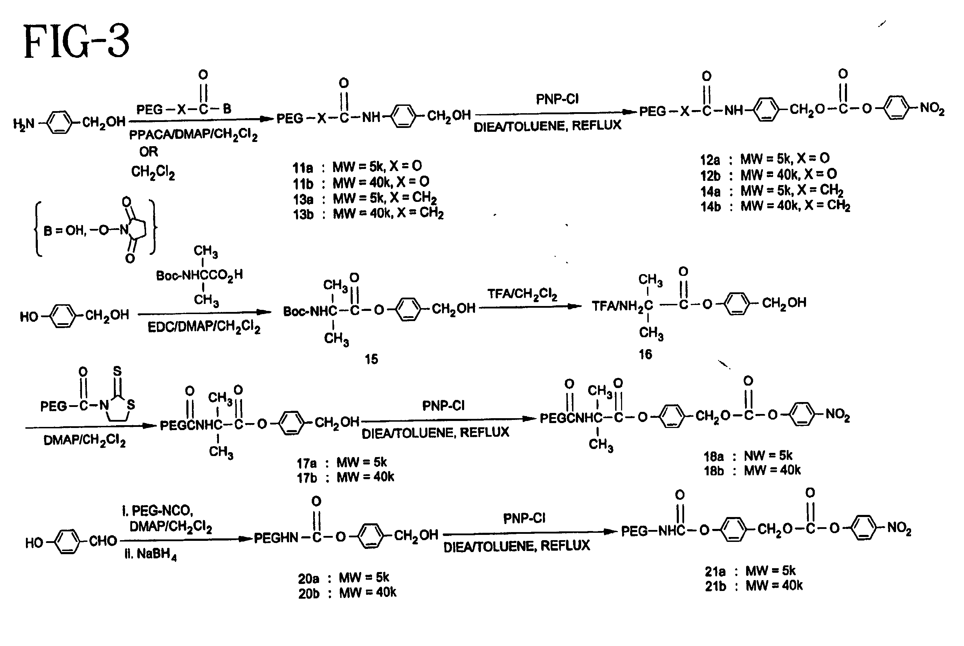

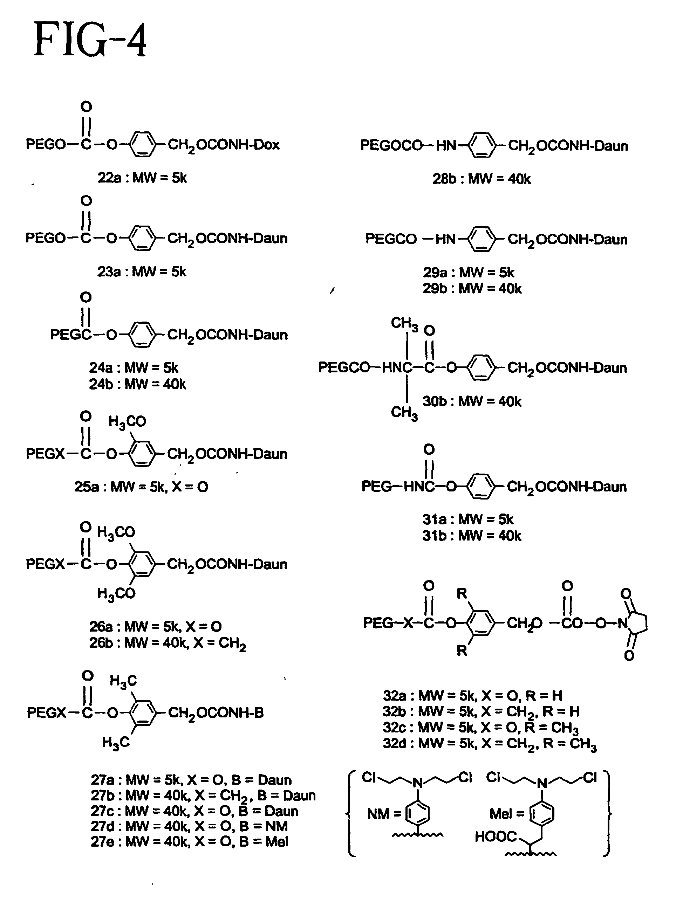

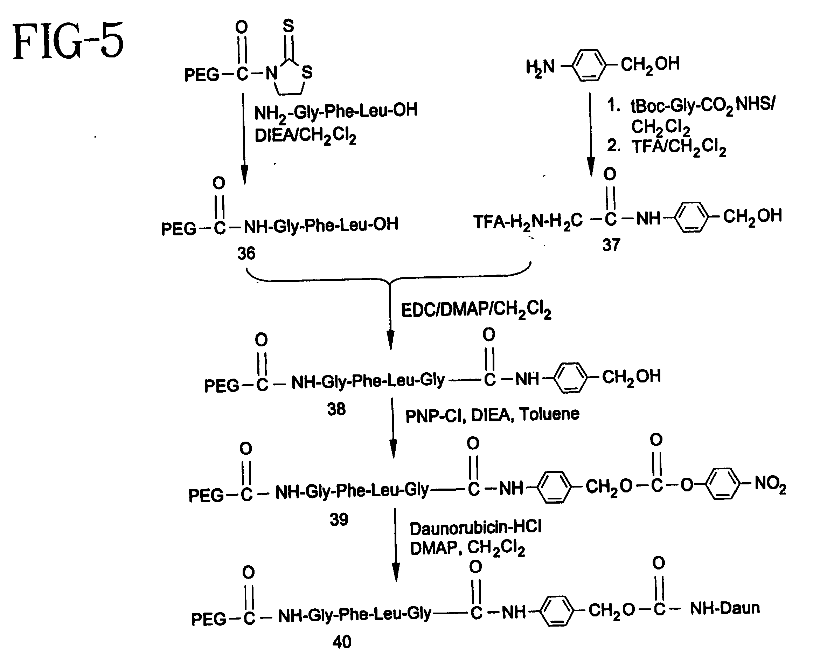

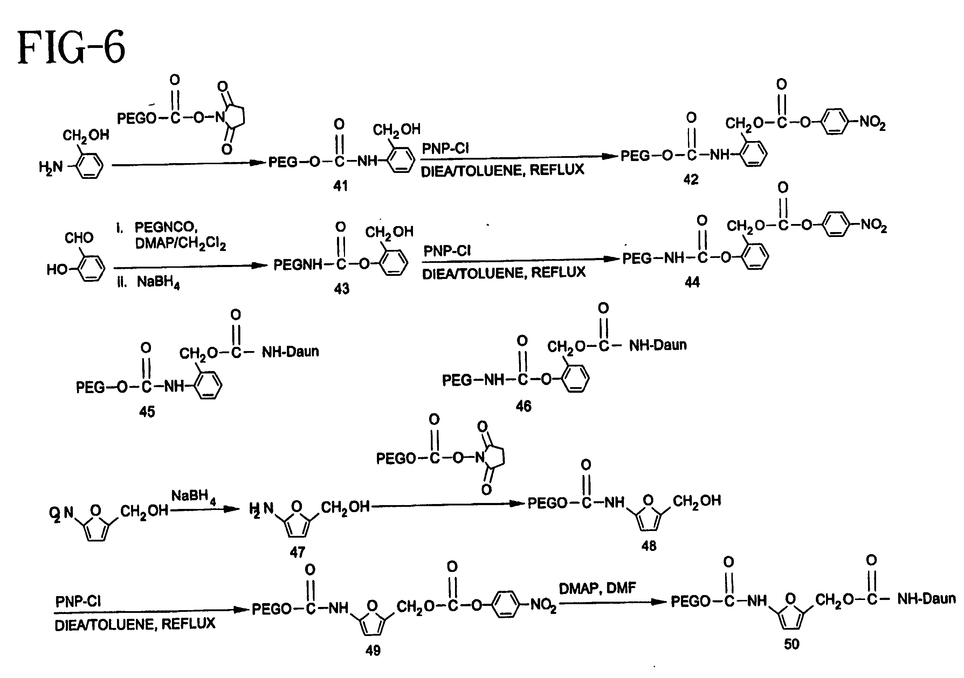

The following examples serve to provide further appreciation of the invention but are not meant in any way to restrict the effective scope of the invention. The compound numbers mentioned in the examples refer to the compounds identified in Figure 2-6.

### Example 1:

### Synthesis of compound (2a): A solution of 10.0 g (2.0 mmol) of

mPEG 5 kDa thiazolidine thione activated carbamate, 0.5 g (4.0 mmol) of 4-hydroxybenzyl alcohol and 0.5 g (4.0 mmol) of 4-(dimethylamino)pyridine (DMAP) in 50 mL of dry methylene chloride was refluxed for 18 hours. The solvent was removed from the reaction mixture by distillation *in vacuo* followed by crystallization of the residue from 2-propanol to yield 9.0 g (87% yield) of alcohol **1a**.
¹³C NMR (67.80 MHz, CDCl₃) δ 58.15, 62.97, 66.86-71.14 (PEG), 120.01, 126.98, 138.97, 149.35, 152.79.

A solution of 5.0 g (1.0 mmol) of **1a** in 75 mL of toluene was azeotroped for 2 hours while removing 25 ml of toluene/water. The reaction mixture was cooled to 30 °C followed by the addition of 0.4 g (2.0 mmol) of 4-nitrophenylchloroformate (PNP-Cl) and 0.26 g (2.0 mmol) of diisopropylethylamine (DIEA). This mixture was stirred for 18 hours at 50-55 °C followed by cooling and removal of the solvent by distillation *in vacuo.* The residue was crystallized from 20% methylene chloride in ethyl ether to yield 3.7 g (70% yield) of product **2a.**
¹³C NMR (67.80 MHz, CDCl₃) δ 58.25, 67.14-71.21(PEG), 120.72, 121.24, 124.58, 129.32, 131.44, 144.71, 150.79, 154.78, 151.63, 152.64.

### Examples 2:

**Synthesis of compound (2b);** Compound **2b** is prepared in a similar manner to compound **2a** using a 40 kDa PEG dithiazolidine thione carbamate in place of the 5 kDa PEG.

### Example 3: (not according to the invention)

**Synthesis of compound (4a):** A solution of 10.0 g (2.0 mmol) of mPEG 5 kDa acid, 1.0 g (8.0 mmol) of 4-hydroxybenzyl alcohol and 1.0 g (8.0 mmol) of DMAP in 100 mL of dry methylene chloride was cooled to 0 °C followed by the addition of 1.0 g (8.0 mmol) of diisopropylcarbodiimide (DIPC). The reaction mixture was allowed to slowly warm to room temperature overnight. The solvent was removed by distillation *in vacuo,* and the residue was crystallized from 2-propanol to yield 8.6 g (83% yield) of product **3a.**
¹³C NMR (67.80 MHz, CDCl₃) δ 57.88, 62.65, 67.54-71.13 (PEG), 120.17, 126.77, 138.85, 148.20, 167.92.

Compound **3a** can also be made using mPEG 5 kDa thiazolidine thione amide in place of the mPEG 5 kDa acid, in the presence ofDMAP and 4-hydroxybenzyl alcohol in methylene chloride.

A solution of 3.0 g (0.58 mmol) of **3a** in 75 mL of toluene was azeotroped for 2 hours while removing 25 mL of toluene/water. The reaction mixture was cooled to 30°C followed by the addition of 0.23 g (1.1 mmol) of PNP-Cl and 0.15 g (1.2 mmol) of DIEA. This mixture was stirred for **18** hours at 50-55 °C followed by cooling and removal of the solvent by distillation *in vacuo.* The residue was crystallized from 20% methylene chloride in ethyl ether to yield 2.4 g (77% yield) of product **4a.**
¹³C NMR (67.80 MHz, CDCl₃) δ 57.90, 67.53-70.92 (PEG), 120.85, 121.06, 124.32, 129.03, 131.23, 144.42, 149.67, 154.52, 151.34, 167.79.

### Example 4: (not according to the invention)

**Synthesis of compound (4b):** A solution of 4.0 g (0.1 mmol) of 40 kDa PEG-dithiazolidine thione amide, 0.26 g (2.1 mmol) of 4-hydroxybenzyl alcohol and 0.25 g (2.1 mmol) of DMAP in 40 mL of dry methylene chloride was refluxed overnight. The solvent was removed by distillation *in vacuo,* and the residue was crystallized from 2-propanol to yield 3.4 g (85% yield) of product **3b.**
¹³C NMR (67.80 MHz, CDCl₃) δ 63.57, 68.36-71.86 (PEG), 120.69, 127.31, 139.15, 149.25, 168.21.

A solution of 3.0 g (0.07 mmol) of **3b** in 140 mL of toluene was azeotroped for 2 hours while removing 40 mL of toluene/water. The reaction mixture was cooled to 30 °C followed by the addition of 0.06 g (0.3 mmol) of PNP-Cl and 0.04 g (0.3 mmol) of DIEA. This mixture was stirred for 18 hours at 50-55 °C followed by cooling and removal of the solvent by distillation *in vacuo.* The residue was crystallized from 20% methylene chloride in ethyl ether to yield 2.4 g (77% yield) of product **4b.**
¹³C NMR (67.80 MHz, CDCl₃) δ 68.47-71.32(PEG) 121.39, 121.47, 124.87, 129.45, 131.96, 145.54, 150.15, 155.01,151.82, 168.19.

### Example 5:

**Synthesis of compound (6a):** A solution of 2.5 g (0.5 mmol) of mPEG 5 kDa thiazolidine thione carbamate, 0.16 g (1.0 mmol) of 4-hydroxy-3,5-dimethylbenzyl alcohol and 0.12 g (1.0 mmol) of DMAP in 50 mL of dry methylene chloride was refluxed for 18 hours. The solvent was removed from the reaction mixture by distillation *in vacuo* followed by crystallization of the residue from 2-propanol to yield 2.2 g (85%yield) of alcohol **5a.**
¹³C NMR (67.80 MHz, CDCl₃) δ 15.10, 57.94, 63.25, 66.96-71.71(PEG), 126.30, 129.19, 138.87, 149.90, 152.12.

A solution of 2.2 g (0.42 mmol) of **5a** in 75 mL of toluene was azeotroped for 2 hours while removing 25 mL of toluene/water. The reaction mixture was cooled to 30 °C followed by the addition of 0.17 g (0.85 mmol) of PNP-Cl and 0.11 g ( 0.85 mmol) of DIEA. This mixture was stirred for 18 hours at 50-55 °C followed by cooling and removal of the solvent by distillation *in vacuo.* The residue was crystallized from 20% methylene chloride in ethyl ether to yield 1.9 g (86%) of product **6a.**
¹³C NMR (67.80 MHz, CDCl₃) δ 15.28, 58.14, 67.32-71.50(PEG), 121.19, 124.58, 128.31, 130.31, 131.65, 145.16, 148.39, 151.73, 152.09, 155.15.

### Example 6: (not according to the invention)

**Synthesis of compound (6b):** Compound **6b** was prepared in a similar manner to compound 6a using a 40 kDa PEG dithiazolidine thione amide in place of the 5 kDa PEG.
¹³C NMR (67.80 MHz, CDCl₃) δ 15.78, 63.86, 68.10, 68.71-71.58 (PEG), 126.71, 129.58, 138.97, 148.39, 152.09, 167.66.

### Examples 7:

**Synthesis of compound (6c):** A solution of 6.0 g (0.15 mmol) of (di-SC)-PEG 40 kDa and 0.6 g (4.0 mmol) of 3,5-dimethyl-4-hydroxy benzyl alcohol in 60 mL of dry methylene chloride was refluxed overnight. The solvent was removed by distillation *in vacuo,* and the residue was crystallized from 2-propanol to yield 5.4 g (90% yield) of product **5c.**
¹³C NMR (67.80 MHz, CDCl₃) δ 15.49, 63.44, 67.06, 68.31, 68.58-70.90 (PEG), 126.53, 129.37, 138.79, 146.78, 152.36.

A solution of 2.0 g (0.05mmol) of 5c in 80 mL of toluene was azeotroped for 2 hours while removing 40 mL of toluene/water. The reaction mixture was cooled to 30 °C followed by the addition of 0.04 g (0.2 mmol) of PNP-Cl and 0.03 g (0.2 mmol) of DIEA. This mixture was stirred for **18** hours at 50-55 °C followed by cooling and removal of the solvent by distillation *in vacuo.* The residue was crystallized from 20% methylene chloride in ethyl ether to yield 1.7 g (85% yield) of product **6c.**
¹³C NMR (67.80 MHz, CDCl₃) δ 15.44, 67.21, 68.23, 68.61-71.26 (PEG), 121.29, 124.65, 128.54, 130.19, 131.41, 144.79, 148.11, 151.73, 152.14, 154.91.

### Example 8: (not according to the invention)

**Synthesis of compound (8a):** A solution of 3.0 g (0.6 mmol) of mPEG 5 kDa thiazolidine thione activated carbamate, 0.24 g (1.3 mmol) of 4-hydroxy-3,5-dimethoxybenzyl alcohol and 0.16 g (1.3 mmol) of DMAP in 50 mL of dry methylene chloride was refluxed for 18 hours. The solvent was removed from the reaction mixture by distillation *in vacuo* followed by crystallization of the residue from 2-propanol to yield 2.8 g (90% yield) of alcohol **7a.**
¹³C NMR (67.80 MHz, CDCl₃) δ 55.64, 57.94, 63.69, 67.09-71.32 (PEG), 103.26, 129.15, 139.97, 151.70, 152, 14.

A solution of 2.5 g (0.5 mmol) of 7**a** in 75 mL of toluene was azeotroped for 2 hours while removing 25 mL of toluene/water. The reaction mixture was cooled to 30 °C followed by the addition of 0.19 g (1.0 mmol) of PNP-Cl and 0.12 g (1.0 mmol) of DIEA. This mixture was stirred for 18 hours at 50-55 °C followed by cooling and removal of the solvent by distillation *in vacuo.* The residue was crystallized from 20% methylene chloride in ethyl ether to yield 2.3 g (88% yield) of **8a.**
¹³C NMR (67.80 MHz, CDCl₃) δ 56.08, 58.32, 67.59-71.63 (PEG), 105.51, 121.29, 124.76, 130.00, 132.53, 145.32, 151.88, 155.22, 152.09, 152.34, 152.35.

### Example 9: (not according to the invention)

**Synthesis of compound (8b):** Compound 7b was prepared in a similar manner to compound 7a using a 40 kDa PEG dithiazolidine thione amide in place of the 5 kDa PEG.
¹³C NMR (67.80 MHz, CDCl₃) δ 55.93, 64.27, 67.97, 68.68-72.04 (PEG), 103.60, 140.09, 152.01, 167.61.

Compound **8b** was prepared in a similar manner to compound **8a** using **7b** in place of **7a.**
¹³C NMR (67.80 MHz, CDCl₃) δ 56.11, 67.98, 68.36-71.47, 105.56, 105.62, 124.79, 132.56, 145.46, 151.93, 152.38, 155.32, 167.46, 167.49.

### Example 10:

**Synthesis of compound (10a):** A solution of 3.0 g (0.6 mmol) of mPEG 5 kDa thiazolidine thione carbamate, 0.2 g (1.3 mmol) of 4-hydroxy-3-methoxybenzyl alcohol and 0.14 g (1.1 mmol) of DMAP in 40 mL of dry methylene chloride was refluxed for 18 hours. The solvent was removed from the reaction mixture by distillation *in vacuo* followed by crystallization of the residue from 2-propanol to yield 2.4 g (77% yield) of product **9a.**
¹³C NMR (67.80 MHz, CDCl₃) δ 54.94, 57.94, 62.99, 66.82-70.97 (PEG), 110.13, 117.54, 120.95, 138.03, 140.38, 150.13, 152.25.

A solution of 2.2 g (0.42 mmol) **of 9a** in 70 mL of toluene was azeotroped for 2 hours while removing 30 mL of toluene/water. The reaction mixture was cooled to 30 °C followed by the addition of 0.20 g (0.9 mmol) of PNP-Cl and 0.11 g (0.9 mmol) of DIEA. This mixture was stirred for 18 hours at 50-55 °C followed by cooling and removal of the solvent by distillation *in vacuo.* The residue was crystallized from 20% methylene chloride in ethyl ether to yield 1.1 g (48% yield) of product **10a.**
¹³C NMR (67.80 MHz, CDCl₃) δ 55.84, 58.32, 67.54-71.62 (PEG), 112.97, 120.51, 121.29, 122.21, 124.75, 133.05, 140.64, 145.29, 151.21 and 155.22, 151.88, 152.51.

### Example 11. (not according to the invention)

**Synthesis of compound (10b):** Compound **10b** is prepared in a similar manner to compound **10a** using a 40 kDa PEG dithiazolidine thione amide in place of the 5 kDa PEG.

### Example 12:

**Synthesis of compound (12b):** A solution of 4.0 g (0.1 mmol) of disuccinimidyl (di-SC)-PEG 40 kDa , 0.1 g (0.8 mmol) of 4-aminobenzyl alcohol, and 0.1 g (0.8 mmol) of DMAP in 30 mL of dry methylene chloride was stirred overnight at room temperature. The solvent was removed by distillation *in vacuo,* and the residue crystallized from 2-propanol to yield 3.7 g (93% yield) of product **11b.**
¹³C NMR (67.80 MHz, CDCl₃) δ 63.64, 63.99, 68.91-71.32 (PEG), 118.57, 127.06, 136.13, 137.20, 153.08.

A solution of 3.0 g (0.07 mmol) of **11b** in 140 mL of toluene was azeotroped for 2 hours while removing 40 mL of toluene/water. The reaction mixture was cooled to 30 °C followed by the addition of 0.06 g (0.3 mmol) of PNP-Cl and 0.04 g (0.3 mmol) of DIEA. This mixture was stirred for 18 hours at 50-55 °C followed by cooling and removal of the solvent by distillation *in vacuo*. The residue was crystallized from 20% methylene chloride in ethyl ether to yield 2.4 g (77% yield) of product **12b.**
¹³C NMR (67.80 MHz, CDCl₃) δ 64.01, 68.60-71.45 (PEG), 118.78, 121.39, 124.86, 127.29, 128.85, 129.19, 139.13, 155.51, 152.09, 153.19.

### Example 13:

**Synthesis of compound (12a):** Compound **12a** is prepared in a similar manner to 12b by using mPEG 5 kDa SC-PEG in place of the 40 kDa SC-PEG.

### Example 14: (not according to the invention)

**Synthesis of compound (14a):** A solution of 5.0 g (1.0 mmol) of mPEG 5 kDa carboxylic acid, 0.6 g (5.0 mmol) of 4-aminobenzyl alcohol, 2.0 mL (3.0 mmol) of a 50% solution of 1-propanephosphonic acid cyclic anhydride (PPACA) in ethyl acetate and 0.4 g (3.0 mmol) of DMAP in 30 mL of dry methylene chloride was stirred for 18 hours at room temperature. The solvent was removed by distillation *in vacuo,* and the residue was crystallized from 2-propanol to yield 8.6 g (83% yield) of product **13a.**
¹³C NMR (67.80 MHz, CDCl₃) δ 58.07, 63:23, 69.31-71.06 (PEG), 118.97, 126.51, 135.82, 136.96, 167.28.

A solution of 3.0 g (0.58 mmol) of **13a** in 75 mL of toluene was azeotroped for 2 hours while removing 25 mL of toluene/water. The reaction mixture was cooled to 30 °C followed by the addition of 0.23 g (1.1 mmol) of PNP-Cl and 0.15 g (1.2 mmol) of DIEA. This mixture was stirred for 18 hours at 50-55 °C followed by cooling and removal of the solvent by distillation *in vacuo.* The residue was crystallized from 20% methylene chloride in ethyl ether to yield 2.6 g (84% yield) of product **14a.**
¹³C NMR (67.80 MHz, CDCl₃) δ 58.45, 69.57-71.4 (PEG), 119.61, 121.37, 124.75, 129.11, 129.42, 137.88, 144.86, 155.04, 151.86, 167:95.

### Example 15: (not according to the invention)

**Synthesis of compound (14b):** A solution of 5.0 g (0.12 mmol) of PEG 40 kDa dicarboxylic acid, 0.15 g (1.2 mmol) of 4-aminobenzyl alcohol, 0.5 mL (0.8 mmol) of a 50% solution of PPACA in ethyl acetate and 0.09 g (0.8 mmol) of DMAP in 100 mL of dry methylene chloride was stirred overnight at room temperature. The solvent was removed by distillation *in vacuo,* and the residue was crystallized from 2-propanol to yield 2.54 g (56% yield) of product **13b.**
¹³C NMR (67.80 MHz, CDCl₃) δ 63.98, 68.99-71.0 (PEG),119.60, 127.01, 136.50, 137.35, 167.48.

A solution of 3.0 g (0.07 mmol) of **13b** in 140 mL of toluene was azeotroped for 2 hours while removing 40 mL of toluene/water. The reaction mixture was cooled to 30 °C followed by the addition of 0.06 g (0.3 mmol) of PNP-Cl and 0.04 g (0.3 mmol) of DIEA. This mixture was stirred for 18 hours at 50-55 °C followed by cooling and removal of the solvent by distillation *in vacuo*. The residue was crystallized from 20% methylene chloride in ethyl ether to yield 2.6 g (84% yield) of product **14b.**
¹³C NMR (67.80 MHz, CDCl₃) δ 69.00-71.97 (PEG), 119.78, 121.31, 124.75, 128.98, 129.84, 138.17, 144.86, 155.38, 151.67, 167.77.

### Example 16: (not according to the invention)

**Synthesis of (18b):** A solution of t-Boc-aminoisobutyric acid (2.0 g, 10 mmol), 2.6 g (21.0 mmol) of 4-hydroxybenzyl alcohol, 4.0 g (21.0 mmol) of 1-ethyl-3-(3-dimethylaminopropyl)-carbodiimide hydrochloride (EDC), and 2.6 g (21.3 mmol) ofDMAP in 100 mL of dry methylene chloride was stirred at room temperature overnight. The solvent was removed by distillation *in vacuo,* and the residue was crystallized from methanol to yield 2.6 g (83% yield) of product **15.**
¹H NMR (270.05 MHz, CDCl₃) δ 1.45 (s, 9H), 1.61 (s, 6H), 4.64 (s, 2H), 7.06 (d, 2H, *J* = 8.1 Hz), 7. 3 5 (d, 2H, *J* = **8**.1 Hz).
¹³C NMR (67.80 MHz, CDCl₃) δ 25.38, 28.30, 56.13, 64.34, 121.39, 127.94, 138.55, 150.37, 154.68, 173.49.

Trifluoroacetic acid (TFA, 2.5 mL) was added to a solution of 1 g (3.2 mmol) of 15 in 5 mL of methylene chloride followed by stirring at room temperature for 30 minutes. Ether was added until the solid precipitated. The solid was filtered and washed thoroughly with ether until all the excess TFA is removed. The TFA salt 16 was dried and used as such in the next step.

A solution of 2.0 g (0.05 mmol) of 40 kDa PEG dithiazolidine thione amide, 0.065 g (0.2 mmol) of **16**, and 0.05 g (0.4 mmol) of DMAP in 30 mL of dry methylene chloride was refluxed for 18 hours. The solvent was removed by distillation *in vacuo,* and the residue was recrystallized from 2-propanol to yield 1.9 g (95% yield) of product **17b.** ¹³C NMR (67.80 MHz, CDCl₃) δ 24.49, 55.24, 63.49, 68.65-71.26 (PEG), 120.85, 127.21, 138.79, 168.99.

Compound **18b** was prepared in a similar manner as **14b** by using **17b** in the place of **13b.**

### Examples 17: (not according to the invention)

**Synthesis of compound (18a):** Compound 18a is prepared in a similar manner as **18b** by using 5 kDa PEG thiazolidine thione amide in the place of 40 kDa PEG.

### Example 18:

**Synthesis of compound (21a):** A solution of 10.0 g (2.0 mmol) of mPEG 5 kDa isocyanate, 0.5 g (4.0 mmol) of 4-hydroxybenzaldehyde and 0.5 g (4.0 mmol) of DMAP in 50 mL of dry methylene chloride was refluxed for 18 hours. The solvent was removed from the reaction mixture by distillation *in vacuo* followed by crystallization of the residue from 2-propanol to yield the aldehyde **19a.**

To a solution of 0.25 g (0.05 mmol) of this aldehyde in 40 mL of methanol at 0 °C was added 6.0 mg (0.15 mmol) of sodium borohydride followed, by stirring for 2 hours. The solvent was removed from the reaction mixture by distillation *in vacuo* followed by dissolving the residue in 30 mL of methylene chloride and washing with dilute aqueous HCl The organic layer was separated and dried over anhydrous sodium sulfate. The solvent was removed by distillation *in vacuo,* and the residue was crystallized from 2-propanol to yield 1.5 g (75% yield) of **20b.**

A solution of 5.0 g (1.0 mmol) of **20a** in 75 mL of toluene was azeotroped for 2 hours while removing 25 mL of toluene/water. The reaction mixture was cooled to 30 °C followed by the addition of 0.4 g (2.0 mmol) of PNP-Cl and 0.26 g (2.0 mmol) of DIEA. This mixture was stirred for 18 hours at 50-55 °C followed by cooling and removal of the solvent by distillation *in vacuo.* The residue was crystallized from 20% methylene chloride in ethyl ether to yield 3.7 g (70% yield) of product **21a.**
¹³C NMR (67.80 MHz, CDCl₃) δ 40.66, 58.58, 68.92-71.54 (PEG), 121.45, 121.57, 124.86, 129.17, 129.51, 130.66, 145.00, 151.32, 151.96, 154.03, 155.12.

### Example 19:

**Synthesis of (21b):** Compound **20b** was prepared in a similar manner as **20a** by using 40 kDa PEG kDa diisocyanate in place of 5k mPEG isocyanate. ¹³C NMR (67.80 MHz, CDCl₃) δ 40.35, 63.44, 67.97-71.45 (PEG), 120.82, 127.05, 137.99, 147.68, 154.13.

Compound **21b** was prepared in a similar manner as **21a** using **20b** in the place of **20a.**
¹³C NMR (67.80 MHz, CDCl₃) δ 40.72, 67.81-71.99 (PEG), 121.53, 121.66, 121.96, 124.68, 124.96, 125.20, 129.63, 130.73, 145.07, 151.37, 152.05, 154.12, 155.181.

### Example 20:

**Synthesis of compound (22a):** A mixture of 0.5 g (0.09 mmol) of **2a,** 65 mg (0.11 mmol) of doxorubicin hydrochloride and 46 mg (0.38 mmol) of DMAP in 10 mL of dry dimethylformamide was stirred at room temperature for 18 hours. To this mixture was added 30 mL of ether. The precipitate was collected by filtration and washed with ether followed by crystallization from 2-propanol to yield 0.38 g (70% yield) of product **22a.**
¹³C NMR (67.80 MHz, CDCl₃) δ 16.38, 29.25, 33.13, 34.93, 42.11, 44.71, 46.58, 56.01, 58.32, 64.76, 64.93, 67.06, 68.06, 68.26, 68.81, 68.99-71.26 (PEG),75.91, 100.32, 110.54, 110.68, 118.06, 119.04, 119.98, 120.37, 120.66, 128.65, 129.16, 132.98, 133.27, 133.91, 134.60, 135.19, 150.09, 152.75, 154.86, 155.56, 160.34, 185.80, 186.10, 213.07.

### Example 21:

**Synthesis of (23a):** A mixture of 0.5 g (0.09 mmol) of **2a,** 65 mg (0.11 mmol) of daunorubicin hydrochloride and 46 mg (0.38 mmol) of DMAP in 10 mL of dry dimethylformamide was stirred at room temperature for 18 hours. To this mixture was added 30 mL of ether. The precipitate was collected by filtration and washed with ether followed by crystallization from 2-propanol to yield 0.44 g (80% yield) of product.
¹³C NMR (67.80 MHz, CDCl₃) δ 16.52, 24.47, 29.69, 32.87, 34.62, 44.97, 46.88, 56.29, 58.58, 65.23, 67.03, 67.30, 68.31, 68.68, 69.39-71.50 (PEG), 76.25, 100.73, 110.73, 110.91, 118.16, 119.31, 120.40, 120.59, 120.90, 128.85, 129.38, 133.88, 134.09, 135.01, 135.35, 153.03, 155.10, 155.38, 156.03, 160.60, 186.16, 186.47, 211.77.

### Example 22: (not according to the invention)

**Synthesis of (24a):** A mixture of 0.5 g (0.09 mmol) of **4a,** 65 mg (0.11 mmol) of daunorubicin hydrochloride and 46 mg (0.38 mmol) ofDMAP in 10 mL of dry dimethylformamide was stirred at room temperature for 18 hours. To this mixture was added 30 mL of ether. The precipitate was collected by filtration and washed with ether followed by crystallization from 2-propanol to yield 0.38 g (75% yield) of product **24a.**
¹³C NMR (67.80 MHz, CDCl₃) δ 16.25, 24.07, 29.10, 32.35, 34.27, 46.62, 55.93, 58.22, 64.87, 66.75, 67.81, 68.24, 68.50, 68.60, 68.83-71.19 (PEG), 75.90, 100.19, 110.34, 110.51, 117.97, 118.91, 119.91, 120.66, 120.87, 128.60, 129.38, 133.66, 133.86, 134.54, 135.06, 154.81, 154.93, 155.62, 160.26, 168.08, 185.71, 185.95, 211.17.

### Example 23: (not according to the invention)

**Synthesis of (24b):** Compound **24b** was prepared in a similar manner to compound **24a** using a 40 kDa MW PEG linker **4b** in place of MW 5 kDa linker **4a.** UV assay for this compound indicated the amount of daunorubicin present is 2.3 %. *In vitro* and *in vivo* results for this compound are set forth in Table 1 below.

### Example 24:

**Synthesis of (25a):** A mixture of 0.5 g (0.09 mmol) of **10a,** 65 mg (0.11 mmol) of daunorubicin hydrochloride and 46 mg (0.38 mmol) of DMAP in 10 mL of dry dimethylformamide was stirred at room temperature for 18 hours. To this mixture was added 30 mL of ether. The precipitate was collected by filtration and washed with ether followed by crystallization from 2-propanol to yield 0.44 g (80% yield) of product. UV assay for this compound indicated the amount of daunorubicin present is 9.2 %.

### Example 25:

**Synthesis of (26a):** A mixture of 0.5 g (0.09 mmol) of **8a**, 65 mg (0.11mmol) of daunorubicin hydrochloride and 46 mg (0.38 mmol) of DMAP in 10 mL of dry dimethylformamide was stirred at room temperature for 18 hours. To this mixture was added 30 mL of ether. The precipitate was collected by filtration and washed with ether followed by crystallization from 2-propanol to yield 0.42 g (81% yield) of product. UV assay for this compound indicated the amount of daunorubicin present is 9.2 %.

### Example 26: (not according to the invention)

**Synthesis of compound (26b):** Compound **26b** was prepared in a similar manner to compound 26a using a 40 kDa PEG linker **8b** in place of the 5 kDaPEG linker **8a.** UV assay for this compound indicated the amount of daunorubicin present is 2.1%.

### Example 27:

**Synthesis of (27a):** A mixture of 0.5 g (0.09 mmol) of **6a,** 65 mg (0.11 mmol) of daunorubicin hydrochloride and 46 mg (0.38 mmol) ofDMAP in 10 mL of dry dimethylformamide was stirred at room temperature for 18 hours. To this mixture was added 30 mL of ether. The precipitate was collected by filtration and washed with ether followed by crystallization from 2-propanol to yield 0.44 g (85% yield) of product. UV assay for this compound indicated the amount of daunorubicin present is 9.2 %.

### Example 28: (not according to the invention)

**Synthesis of compound (27b):** Compound **27b** was prepared in a similar manner to compound **27a** using a 40 kDa PEG linker **6b** in place of 5 kDaPEG linker **6a.** UV assay for this compound indicated the amount of daunorubicin present is 2.3 %. *In vitro* and *in vivo* results for this compound are set forth in Table 1 below.

### Example 29:

**Synthesis of compound (27c):** Compound **27c** was prepared in a similar manner to compound **27a** using a 40 kDa PEG linker **6c** in place of 5 kDaPEG linker **6a.** UV assay for this compound indicated the amount of daunorubicin present is 2.5 %.

### Example 30:

Synthesis of compound (27d): A mixture of 1.5 g (0.037 mmol) of **6c,** 50 mg (0.19 mmol) of *p*-amino-(*N,N*-di-2-chloroethyl)aniline hydrochloride (synthesized using a modified procedure of Edwards et al. Cytotoxic Compounds. Part XVII. *o*-, *m*-, and *p*-(Bis-2-chloroethylamino)phenol, *p*-[*N-*(2-Chloethyl)methylamino]phenol, *N,N-*Bis-2-chloroehyl-*p*-phenylenediamine, and *N,N* Bis-2-chloroethyl-*N*'-methyl-*p-*phenylenediamine as Sources of Biologically Active Carbamates. JCS Perkin I, 1973, 2397.), and 50 mg (0.41 mmol) of DMAP in 15 mL of anhydrous dimethylformamide was stirred at room temperature for 30 minutes and 15 mL of anhydrous dichloromethane was added. The reaction solution was stirred at room temperature overnight and concentrated *in vacuo.* The residue was recrystallized from 2-propanol to give 1.43 g (95 %) of **27d.**
¹³C NMR (67.80 MHz, CDCl₃) δ 15.15, 39.83, 52.85, 62.05, 64.75, 66.83-70.45 (PEG), 77.19, 111.95, 120.15, 127.58,128.73, 129.38, 133.62, 141.35, 147.12, 151.91, 153.00.

### Example 31:

**Synthesis-of compound (27e):** DIEA (0.15 mL, 0.86 mmol) was added to a mixture of 1.0 g (0.025 mmol) of **6c** and 60 mg (0.20 mmol) of melphalan in 15 mL of anhydrous dimethylformamide and the mixture was stirred at room temperature for 30 minutes. Anhydrous dichloromethane (5 mL) was added and the reaction solution was stirred at room temperature overnight and concentrated *in vacuo.* The residue was recrystallized from 2-propanol to give 0.85 g (85 %) of **27e.**
¹³C NMR (67.80 MHz, CDCl₃) δ 15.06, 36.71, 39.63, 52.43, 54.60, 64.01, 66.77-70.43 (PEG), 111.01, 124.26, 127.44, 129.27, 129.73, 134.18, 144.03, 147.03, 151.83, 154.52, 171.68.

### Examples 32:

**Synthesis of (28b):** A mixture of 1.0 g (0.025 mmol) of **12b,** 65 mg (0.11 mmol) of daunorubicin hydrochloride and 46 mg (0.38 mmol) of DMAP in 10 mL of dry dimethylformamide was stirred at room temperature for 18 hours. To this mixture is added 30 mL of ether. The precipitate was collected by filtration and washed with ether followed by crystallization from 2-propanol to yield 0.88 g (88% yield) of **28b.** UV assay for this compound indicated the amount of daunorubicin present is 2.2%. *In vitro* and *in vivo* results for this compound are set forth in Table 1 below.

### Example 33:

**Synthesis of (29a):** A mixture of 0.5 g (0.09 mmol) of **14a,** 65 mg (0.11 mmol) of daunorubicin hydrochloride and 46 mg (0.38 mmol) ofDMAP in 10 mL of dry dimethylformamide was stirred at room temperature for 18 hours. To this mixture was added 30 mL of ether. The precipitate was collected by filtration and washed with ether followed by crystallization from 2-propanol to yield 0.44 g (80% yield) of product.
¹³C NMR (67.80 MHz, CDCl₃) δ 16.20, 23.99, 29.04, 32.29, 34.21, 46.55, 55.84, 58.12, 65.14, 68.14, 68.57, 68.76, 68.81, 69.31-73.37 (PEG), 75.78, 100.11, 110.21, 110.38, 117.93, 118.81, 119.00, 119.25, 119.77, 128.05, 128.48, 131.69, 133.57, 134.39, 134.99, 154.86, 155.53, 160.16, 167.46, 185.58, 185.77, 211.09.

### Example 34:

**Synthesis of compound (29b):** Compound **29b** was prepared in a similar manner to compound **29a** using a 40 kDa PEG linker **14b** in place of the 5 kDaPEG linker **14a.** UV assay for this compound indicated the amount of daunorubicin present is 2.1%.

### Example 35: (Not according to the invention)

**Synthesis of (30b):** A mixture of 1.0 g 0.025 mmol) of **18b,** 65 mg (0.11 mmol) of daunorubicin hydrochloride and 46 mg (0.38 mmol) of DMAP in 10 mL of dry dimethylformamide is stirred at room temperature for 18 hours. To this mixture was added 30 mL of ether. The precipitate was collected by filtration and washed with ether followed by crystallization from 2-propanol to yield 0.8 g (80% yield) of **30b.** UV assay for this compound indicated the amount of daunorubicin present is 1.8%*. In vitro* and *in vivo* results for this compound are set forth in Table 1 below.

### Example 36:

**Synthesis of compound (31a):** A mixture of 0.5 g (0.09 mmol) **of 21a,** 65 mg (0.11 mmol) of daunorubicin hydrochloride and 46 mg (0.38 mmol) of DMAP in 10 mL of dry dimethylformamide was stirred at room temperature for 18 hours. To this mixture is added 30 mL of ether. The precipitate was collected by filtration and washed with ether followed by crystallization from 2-propanol to yield 0.44 g (80% yield) **of 31 a.**

### Example 37:

**Synthesis of compound (31b):** Compound **31b** was prepared in a similar manner to compound **31a** using a 40 kDa PEG linker **21b** in place of the 5 kDa linker **21a.** UV assay for this compound indicated the amount of daunorubicin present is 2.6%. *In vitro* and *in vivo* results for this compound are set forth in Table 1 below.

### Example 38:

**Synthesis of compound (32a):** To a solution of 2 g (0.4 mmol) of **1a** and 0.2 g (0.8 mmol) of *N,N*-disuccinimidyl carbonate in 25 mL of anhydrous methylene chloride was added 30 µL (0.4 mmol) of anhydrous pyridine at 0 °C under nitrogen atmosphere and the solution was stirred overnight at 4 °C. The product was precipitated by the addition of 300 mL of ether. The solid obtained was recrystallized from methylene chloride/ether to give 1.6 g (80%) of the product **32C** as a white solid.
¹³C NMR (67.80 MHz, CDCl₃) δ 24.8, 58.3, 67.2-71.3 (PEG), 120.8, 129.2, 130.6, 150.9, 151.0, 152.6, 168.2.

### Example 39: (not according to invention)

**Synthesis of compound (32b):** Compound 32b was prepared in a similar manner to **32a** starting from **3a.**
¹³C NMR (67.80 MHz, CDCl₃) δ 25.0, 58.5, 67.8-71.8 (PEG), 121.4, 129.5, 130.7, 150.3, 151.1, 168:3, 168.4.

### Example 40:

**Synthesis of compound (32c):** Compound **32c** was prepared in a similar manner to **32a** starting from **5a.**
¹³C NMR (67.80 MHz, CDCl₃) δ 16.0, 25.0, 58.6, 67.8-71.8 (PEG), 128.6, 130.4, 130.7, 149.9, 151.2,167.8, 168.3.

### Example 41: (not according to the invention)

**Synthesis of compound (32d):** Compound **32d** was prepared in a similar manner to **32a** starting from **5d.**
¹³C NMR (67.80 MHz, CDCl₃) δ 15.2, 24.6, 58.1, 67.0-71.2 (PEG), 128.1, 130.0, 130.4, 148.0, 150.8, 151.8, 168.1.

### Example 42:

**Conjugation of compound 2a or 32a to (L)-asparaginase: synthesis of compounds:** PEG linker **2a** or **32a** (450 mg, 0.084 mmol, 317 eq) was added to native (L)-asparaginase (37.5 mg, 416 µL, 0.00027 mmol) in 3 mL of sodium phosphate buffer (0.1 M, pH 7.8) with gentle stirring. The solution was stirred at 30 °C for 30 minutes. A GPC column (Zorbax GF-450) was used to monitor PEG conjugation: The PEG-Asp conjugate had a retention time of 8.5 min. At the end of the reaction (as evidenced by the absence of native enzyme), the mixture was diluted with 12 mL of formulation buffer (0.05 M sodium phosphate, 0.85% sodium chloride, pH 7.3) and diafiltered with a Centriprep concentrator (Amicon) having a molecular weight cut-off of 50,000 daltons to remove the unreacted PEG. Dialfiltration was continued as needed at 4 °C until no more free PEG was detected by mixing equal amount of filtrate and 0.1 % PMA (polymethacrylic acid in 0.1 M HCl).

Compound 33 was not stable in basic buffer solution for prolonged periods of time, therefore the solution was lyophilized and **33** stored in the freezer (-20 °C). After 15 days of storage in this manner, GPC analysis indicated less than 0.8% decomposition. The specific activity of freshly prepared **33** was found to be 137 IU/mg (native asparaginase = 217 IU/mg). Protein modification of asparaginase with SS-PEG (a permanent linker) using a procedure corresponding to that described in the aforementioned U.S. Patent No. 4,179,337 gave a similar activity of 120 IU/mg. A TNBS assay was used to calculate the percentage modification of the protein, and the Biuret assay was used to check the protein concentration.

### Example 43:

**Kinetics of hydrolysis of PEG conjugate of (L)-asparaginase (33) in rat plasma and buffer:** The rate of hydrolysis of compound **33** in rat plasma was measured using a GPC column (Zorbax GF-450) and was found to have a half life of 82 minutes. *In vitro* kinetics were done and the half life was determined to be 10±2 hours in phosphate buffer (pH 7.4).

### Example 44:

**Synthesis of (34), a protein hybrid: conjugation of (33) with SS-PEG (a permanent linker):** PEG linker **2a** (393 mg, 0.073 mmol, 70 eq) was reacted with native (L)-asparaginase (150 mg, 1.664 mL, 0.00106 mmol) in 30 mL of sodium phosphate buffer (0.1 M, pH 7.8) as described in Example 36 at 30 °C for 15 minutes to provide a solution of **33**, followed by the addition of SS-PEG (1.272 g, 0.245 mmol, 230 eq). The reaction solution was stirred for another 15 minutes. The pH of the reaction mixture was maintained at 7.8 with 0.5 M sodium hydroxide. The reaction was diluted with 30 mL of sterile water and diafiltered using a Centriprep concentrator (Amicon) having a molecular weight cut-off of 50,000 daltons to remove any unreacted PEG. Dialfiltration was continued as needed at 4 °C until no more free PEG was detected by mixing equal amount of filtrate and 0.1 % PMA (polymethacrylic acid in 0.1 M HCl). A GPC column (Zorbax GF-450) was used to follow the course of the reaction. The final solution of **34** was lyophilized and stored in the freezer.

### Example 45:

**Demonstration of selective removal of reversible PEG linker (2a) from the hybrid (34): Generation of a permanently modified asparaginase, compounds (35):** 100 mg of **34** is dissoved in 30 mL of pH 7.8 phosphate buffer and stirred at 30 °C overnight. This solution is diluted with 30 mL of sterile water, and diafiltered with a Centriprep concentrator (Amicon) having a molecular weight cut off of 50,000 Daltons to remove free PEG which was formed by selective cleavage of the conjugates formed from the PEG**-2a** linker. The solution now contains only SS-PEG conjugated asparaginase **(35).** Thus, the revesible linker is hydrolyzed, leaving only the relatively perminantly bonded PEG attached to the asparaginase.

### Example 46: (not according to the invention)

**Synthesis of compound (36):** A mixture of 6 g (0.15 mmol) of 40 kDa PEG dithiazolidine thione amide, 150.9 mg (0.45 mmol) of tripeptide Gly-Phe-Leu, and 76 mg (0.6 mmol) of DIEA in anhydrous methylene chloride was stirred for 18 hours. The reaction mixture was washed with 0.1 N HCl (2 x 5 mL), followed by water (5 mL) and dried over anhydrous magnesium sulfate. The solvent was removed under reduced pressure to yield a solid that was recrystallized from 2-propanol to give 4.9 g (80%) of **36.**
¹³C NMR (67.80 MHz, CDCl₃) δ 21.41, 22.17, 24.21, 36.94, 40.66, 42.31, 50.42, 53.86, 70.64-72.22 (PEG), 126.14, 127.87, 128.73, 136.31, 168.42, 169.91, 170.28, 172.21.

### Example 47: (not according to the invention)

**synthesis of compound (37):** A solution of 1.1 g (4.04 mmol) of *t*-Boc-glycine *N-*hydroxysuccinimide ester and 1 g (8.12 mmol) of 4-aminobenzyl alcohol in 15 mL of methylene chloride was stirred at room temperature for 18 hours. The reaction mixture was filtered to remove the precipitated solid (byproduct NHS) and the filtrate was washed with 0.1 N HCl (2 x 5 mL), followed by water (5 mL) and dried. The solvent was removed under reduced pressure to yield a residue that was triturated with ether to give 900 mg (75%) of the pure t-Boc-glycine amide of 4-aminobenzyl alcohol.
¹³C NMR (67.80 MHz, CDCl₃) δ: 28.27, 44.87, 64.45, 80.50, 120.19, 127.66, 136.73, 137.01, 156.55,168.24.

TFA (2.5 mL) was added to a solution of 500 mg (1.78 mmol) of t-Boc-glycine amide of 4-aminobenzyl alcohol in methylene chloride (5 mL), and the solution was stirred for 30 minutes at room temperature. Anhydrous diethylether (50 mL) was added to precipitate the solid that was filtered, washed thoroughly with ether until all the TFA is washed, and dried to give 300 mg (60%) of the **37** as a TFA salt.
¹³C NMR (67.80 MHz, DMSO-d₆) δ: 41.01, 62.58, 119.01,127.22, 136.79, 138.13, 164.62.

### Example 48: (not according to the invention)

**Synthesis of compound (38):** To a solution of 1 g (0.025 mmol) of **36** and 30 mg (0.10 mmol) of **37** in 10 mL of methylene chloride at 0 °C is added 19.2 mg (0.1 mmol) of EDC and 25 mg (0.2 mmol) of DMAP and the mixture is stirred for 3 hours at 0 °C, followed by 18 h at room temperature. The solvent is removed under reduced pressure and the solid obtained is recrystallized from 2-propanol to give the product **38.**

### Example 49: (not according to the invention)

**Synthesis of compound (39):** A solution of 3.0 g (0.075 mmol) of **38** in 140 mL of toluene is azeotroped for 2 hours while removing 40 mL of toluene/water. The reaction mixture is cooled to 30 °C followed by the addition of 0.06 g (0.3 mmol) of PNP-Cl and 0.04 g (0.3 mmol) of DIEA. The reaction mixture is stirred for 18 hours at 50-55 °C followed by cooling and removal of the solvent by distillation *in vacuo.* The residue is crystallized from 20% methylene chloride in ethyl ether to yield **39**.

### Example 50: (not according to the invention)

**Synthesis of compound (40):** A mixture of 0.8 g (0.02 mmol) of **39**, 45 mg (0.1 mmol) of daunorubicin hydrochloride, and 32 mg (0.26 mmol) ofDMAP in 10 mL of dry dimethylformamide is stirred at room temperature for 18 hours. To this mixture is added 30 mL of ether. The precipitate is collected by filtration and washed with ether followed by crystallization from 2-propanol to yield the product **40**.

### Example 51:

**Synthesis of compound (42):** A solution of 4.0 g (0.1 mmol) of (di-SC) PEG 40 kDa and 0.1 g (0.8 mmol) of 2-aminobenzyl alcohol in 30 mL of dry methylene chloride was refluxed overnight. The solvent was removed by distillation *in vacuo,* and the residue was crystallized from 2-propanol to yield the product **41.**

A solution of 3.0 g (0.07 mmol) of **41** in 140 mL of toluene is azeotroped for 2 hours while removing 40 mL of toluene/water. The reaction mixture is cooled to 30 °C followed by the addition of 0.06 g (0.3 mmol) of PNP-Cl and 0.04 g (0.3 mmol) of DIEA. This mixture is stirred for 18 hours at 50-55 °C followed by cooling and removal of the solvent by distillation *in vacuo*. The residue is crystallized from 20% methylene chloride in ether to yield the product **42.**

### Example 52:

**Synthesis of compound (44):** A solution of 4.0 g (0.1 mmol) of 40 kDa PEG isocyanate, 0.1g (0.8 mmol) of 2-hydroxybenzaldehyde, and 0.1 g (0.8 mmol) of DMAP in 50 mL of dry methylene chloride is refluxed for 18 hours. The solvent is removed from the reaction mixture by distillation *in vacuo* followed by crystallization of the residue from 2-propanol to yield the aldehyde. NaBH₄ reduction of the aldehyde product in methanol gives the corresponding benzyl alcohol **43.**

A solution of 3.0 g (0.07 mmol) of **43** in 140 mL of toluene is azeotroped for 2 hours while removing 40 mL of toluene/water. The reaction mixture is cooled to 30 °C followed by the addition of 0.06 g (0.3 mmol) of PNP-Cl and 0.04 g (0.3 mmol) of DIEA. This mixture is stirred for 18 hours at 50-55 °C followed by cooling and removal of the solvent by distillation *in vacuo.* The residue is crystallized from 20% methylene chloride in ether to yield the product **44.**

### Example 53:

**syntheses of compound (45):** A mixture of 0.8 g (0.02 mmol) of **42,** 45 mg (0.1 mmol) of daunorubicin hydrochloride and 32 mg (0.26 mmol) of DMAP in 10 mL of dry dimethylformamide is stirred at room temperature for 18 hours. To this mixture is added 30 mL of ether. The precipitate is collected by filtration and washed with ether followed by crystallization from 2-propanol to yield the product **45.**

### Example 54:

**Synthesis of compound (46):** A mixture of 0.8 g (0.02 mmol) of **44,** 45 mg (0.1 mmol) of daunorubicin hydrochloride and 32 mg (0.26 mmol) of DMAP in 10 mL of dry dimethylformamide is stirred at room temperature for 18 hours. To this mixture is added 30 mL of ether. The precipitate is collected by filtration and washed with ether followed by crystallization from 2-propanol to yield the product **46.**

### Example 55: (not according to the invention)

**Synthesis of compound (47):** A mixture of 160 mg (4.1 mmol) of sodium borohydride and 0.2 g (1.4 mmol) of nitrofuranylmethanol in 20 mL of 2-propanol is stirred at room temperature for 16 hours and the suspension was filtered through Celite. The filtrate is concentrated *in vacuo* to give a crude product 47 which is used for next step without further purification.

### Example 56: (not according to the invention)

**Synthesis of compound (48):** A solution of 4.0 g (0.1 mmol) of (di-SC)-PEG 40 kDa and 0.09 g (0.8 mmol) of **47** in 30 mL of dry methylene chloride is refluxed overnight. The solvent is removed by distillation *in vacuo,* and the residue is recrystallized from 2-propanol to yield the product **48.**

### Example 57: (not according to the invention

**Synthesis of compound (49):** A solution of 3.0 g (0.07 mmol) of **48** in 140 mL of toluene is azeotroped for 2 hours while removing 40 mL of toluene/water. The reaction mixture is cooled to 30 °C followed by the addition of 0.06 g (0.3 mmol) of PNP-Cl and 0.04 g (0.3 mmol) of DIEA. This mixture is stirred for 18 hours at 50-55 °C followed by cooling and removal of the solvent by distillation *in vacuo.* The residue is crystallized from 20% methylene chloride in ether to yield the product **49.**

### Example-58: (not according to the invention)

**Synthesis of compound (50):** A mixture of 0.8 g (0.02 mmol) of **49,** 45 mg (0.1 mmol) of daunorubicin hydrochloride and 32 mg (0.26 mmol) ofDMAP in 10 mL of dry dimethylformamide is stirred at room temperature for 18 hours. To this mixture is added 30 mL of ether. The precipitate is collected by filtration and washed with ether followed by crystallization from 2-propanol to yield the product **50.**

**Table 1. In Vitro and In Vivo Results of PEG 40kDa-Daunorubicin Prodrugs**

| **Compound #** | **t_{1/2} (h) pH 7.4** | **t_{1/2} (h) rat plasma** | **IC₅₀ (nM) P388/O** | **M109^{a} % T/C** | **SKOV3^{b} % T/C** |
|---|---|---|---|---|---|
| **Daunorubicin HCl** | - | - | 2.3 | 68.0^{c} | 35.2 |
| **Esters** | | | | | |
| **24b** | >24 | 0.4 | 8 | 92.8 | - |
| **27b** | >48 | 1.9 | 55 | 90.3 | - |
| **30b** | >48 | 1.3 | 18 | 89.6 | - |
| **Carbamates** | | | | | |
| **31b** | >48 | 4.1 | 15 | 84.1 | 7.6 |
| **28b** | >48 | >24 | 415 | 75.3 | 51.7 |

| | | | | | |
|---|---|---|---|---|---|
| a : 3 mg/kg/dose of active daunorubicin was given i.p. in balb/c mice bearing S.C. Madison 109 Lung Carcinoma at 1 & 4 days after inoculation. The median tumor volume of treatment and control groups were measured and compared when the control group's median tumor volume reached approximately 2000 mm3. b : 3 mg/kg/dose of active daunorubicin was administered intravenously in nude mice bearing a human ovarian carcinoma xenografts at 1, 5 & 9 days after inoculation. The median tumor volume of treatment and control groups were measured and compared when the control group's median tumor volume reached approximately 1000 mm3. c William C. Rose. Evaluation of Madison 109 Lung Carcinoma as a Model for Screening Antitumor Drugs. Cancer Treatment Reports, 1981, 65, 299. | | | | | |

## Claims

1. A compound selected from the group consisting of: 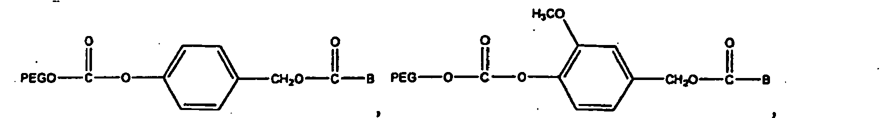 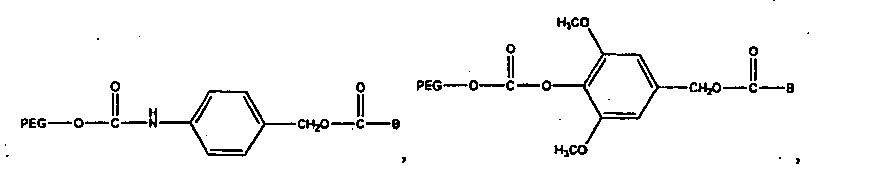 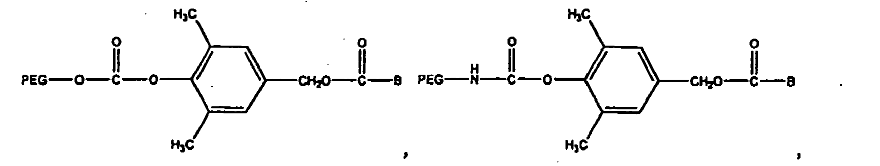 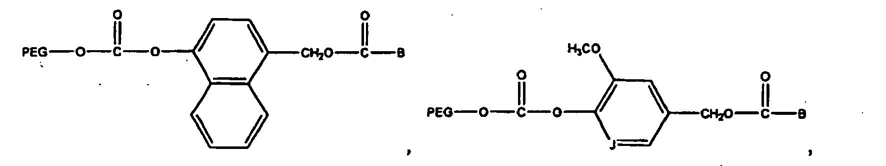 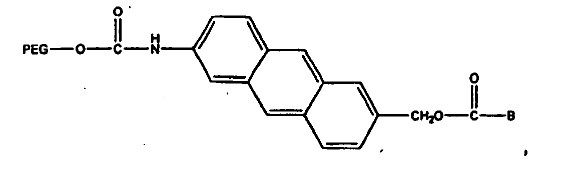 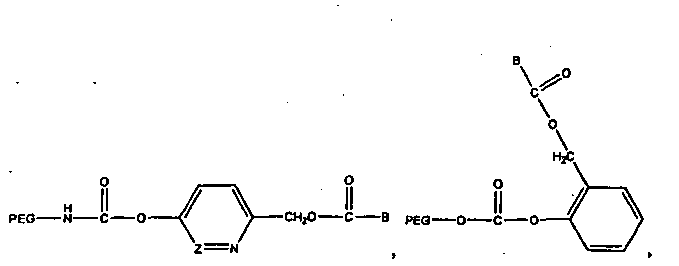 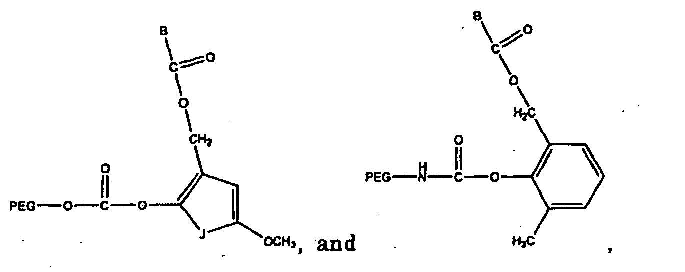 wherein
PEG is polyethylene glycol;
B is a residue of an amine-containing moiety or a residue of a hydroxyl-containing moiety linked to C(=O) at the amine or hydroxyl moiety;
J is O, S, or NR₁₃;
Z is CR₁₃ or NR₁₃; and
R₁₃ is independently-selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, and substituted C₁₋₆ heteroalkyls.

2. The compound of claim 1, wherein B is a residue of a hydroxyl-containing compound.

3. The compound of claim 1, wherein B is a residue of an amine-containing compound.

4. The compound of claim 1, wherein said PEG has an average molecular weight of from about 2,000 to about 100,000 daltons.

5. The compound of claim 4, wherein said PEG has an average molecular weight of from 5,000 to about 40,000 daltons.

6. A prodrug compound of claim 1, method for treatment of a mammal by therapy.

## Patentansprüche

1. Verbindung, ausgewählt aus der Gruppe, bestehend aus: 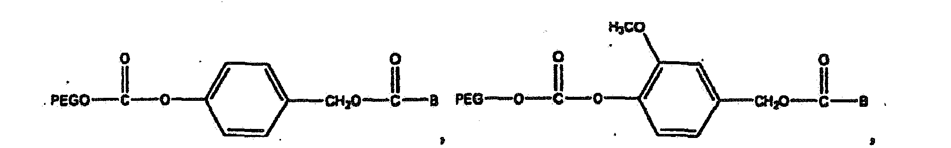 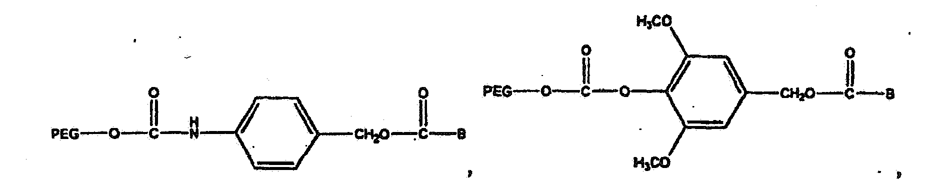 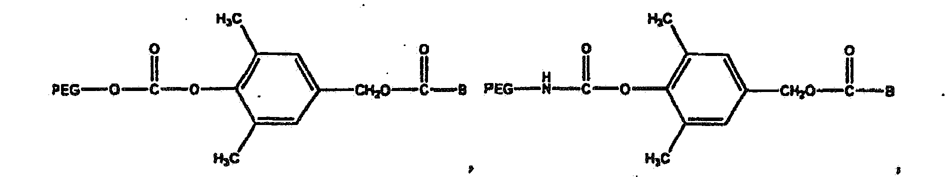 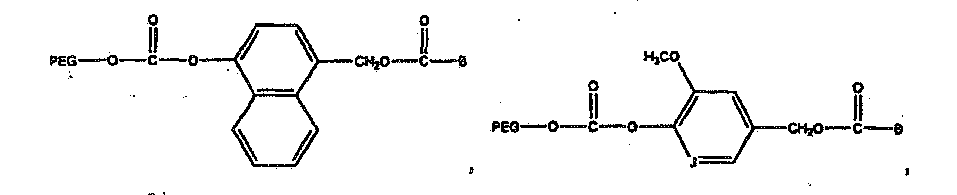 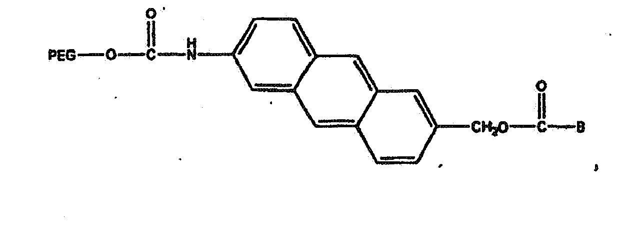 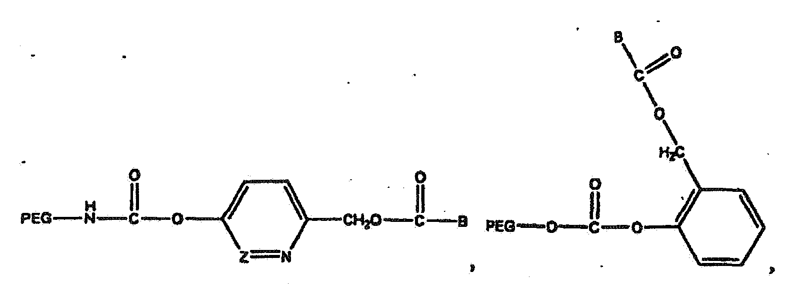 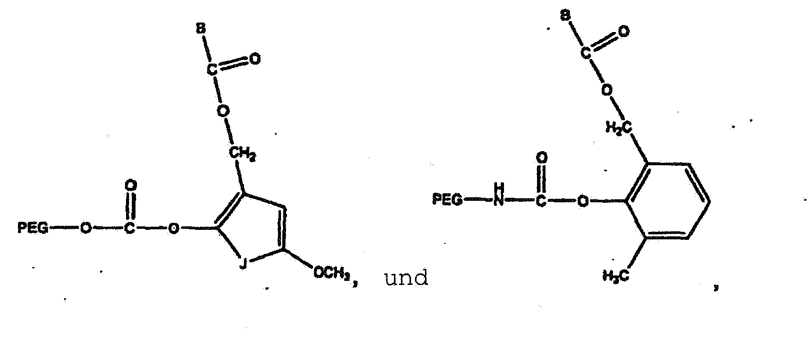 worin:
PEG Polyethylenglykol ist;
B ein Rest einer aminhaltigen Gruppe oder ein Rest einer hydroxylhaltigen Gruppe ist, die an C(=O) an der Amin- oder Hydroxylgruppe gebunden ist;
J O, S oder NR₁₃ ist;
Z CR₁₃ oder NR₁₃ ist; und
R₁₃ unabhängig ausgewählt ist aus der Gruppe, bestehend aus Wasserstoff, C₁₋₆-Alkyl, C₃₋₁₂-verzweigtem Alkyl, C₃₋₈-Cycloalkyl, C₁₋₆-substituiertem Alkyl, C₃₋₈-substituiertem Cycloalkyl, Aryl, substituiertem Aryl, Aralkyl, C₁₋₆-Heteroalkyl und substituiertem C₁₋₆-Heteroalkyl.

2. Verbindung von Anspruch 1, in der B ein Rest einer hydroxylhaltigen Verbindung ist.

3. Verbindung von Anspruch 1, in der B ein Rest einer aminhaltigen Verbindung ist.

4. Verbindung von Anspruch 1, in der das PEG ein mittleres Molekulargewicht von etwa 2.000 bis etwa 100.000 Dalton hat.

5. Verbindung von Anspruch 4, in der das PEG ein mittleres Molekulargewicht von 5.000 bis etwa 40.000 Dalton hat.

6. Prodrug-Verbindung von Anspruch 1 für die Verwendung in einem Verfahren zur Behandlung eines Säugers durch Therapie.

## Revendications

1. Composé choisi dans le groupe consistant en : 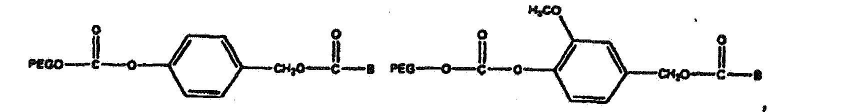 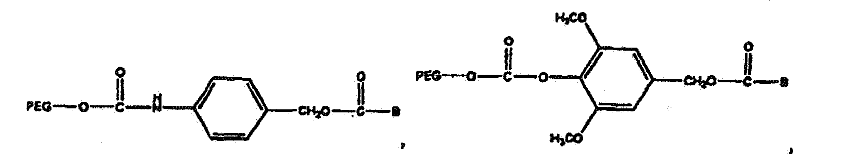 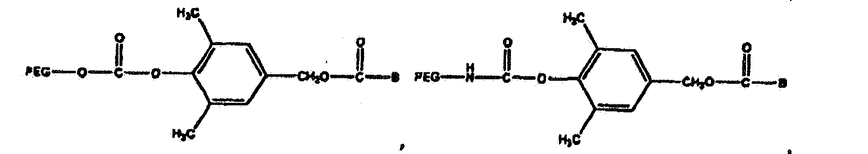 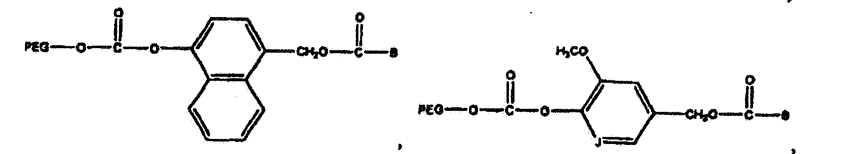 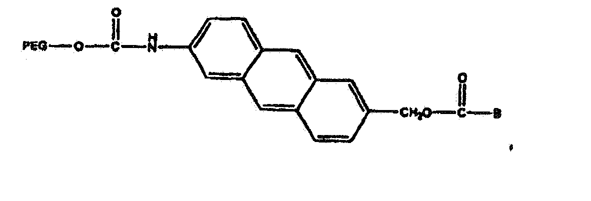 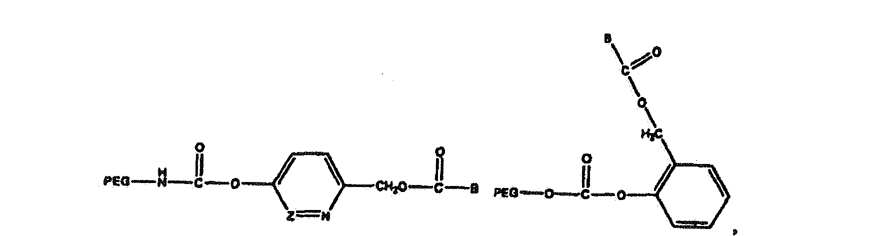 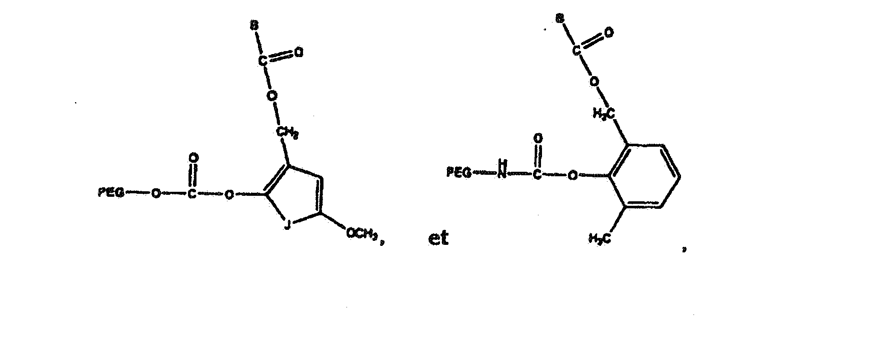 où
PEG est le polyéthyléneglycol ;
B est un résidu d'un groupement contenant une ou des amines ou un résidu d'un groupement contenant un ou des hydroxyles lié à C(=O) au niveau du groupement amine ou hydroxyle ;
J est O, S ou NR₁₃ ;
Z est CR₁₃ ou NR₁₃ ; et
R₁₃ est choisi indépendamment dans le groupe consistant en l'hydrogène, les C₁₋₆-alkyles, les C₃₋₁₂-alkyles ramifiés, les C₃₋₈-cycloalkyles, les C₁₋₆-alkyles substitués, les C₃₋₈-cycloalkyles substitués, les aryles, les aryles substitués, les aralkyles, les C₁₋₆-hétéroalkyles et les C₁₋₆-hétéroalkyles substitués.

2. Composé selon la revendication 1 où B est un résidu d'un composé contenant un ou des hydroxyles.

3. Composé selon la revendication 1 où B est un résidu d'un composé contenant une ou des amines.

4. Composé selon la revendication 1 où ledit PEG a une masse moléculaire moyenne d'environ 2 000 à environ 100 000 daltons.

5. Composé selon la revendication 4 où ledit PEG a une masse moléculaire moyenne de 5 000 à environ 40 000 daltons.

6. Composé promédicament selon la revendication 1 destiné à être utilisé dans un procédé pour le traitement d'un mammifère par thérapie.
